(19)

Europäisches
Patentamt
European
Patent Office
Office européen
des brevets

(11) **EP 3 586 141 B1**

(12) **EUROPEAN PATENT SPECIFICATION**

(45) Date of publication and mention
of the grant of the patent:
**18.01.2023 Bulletin 2023/03**

(21) Application number: **18705960.5**

(22) Date of filing: **27.02.2018**

(51) International Patent Classification (IPC):
***G01N 33/576*** *(2006.01)*     ***G01N 33/68*** *(2006.01)*

(52) Cooperative Patent Classification (CPC):
**G01N 33/576; G01N 33/6893;** G01N 2800/085;
G01N 2800/52; G01N 2800/56

(86) International application number:
**PCT/EP2018/054809**

(87) International publication number:
**WO 2018/154140 (30.08.2018 Gazette 2018/35)**

(54) **NON-INVASIVE DIAGNOSIS OF FIBROSING NON-ALCOHOLIC STEATOHEPATITIS (NASH)**

NICHT-INVASIVE DIAGNOSE DER FIBROSIERENDEN NICHTALKOHOLISCHEN
STEATOHEPATITIS (NASH)

DIAGNOSTIC NON-INVASIF DE LA STÉATOHÉPATITE NON ALCOOLIQUE (NASH) FIBROSANTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **27.02.2017   US 201762464106 P**
**24.05.2017   EP 17305615**

(43) Date of publication of application:
**01.01.2020 Bulletin 2020/01**

(73) Proprietors:
• **Université d'Angers**
**49000 Angers (FR)**
• **Centre Hospitalier Universitaire d'Angers**
**49100 Angers (FR)**

(72) Inventor: **BOURSIER, Jérôme**
**49320 Brissac-Quincé (FR)**

(74) Representative: **Icosa**
**83 avenue Denfert-Rochereau**
**75014 Paris (FR)**

(56) References cited:
**WO-A1-2013/049509     WO-A1-2014/049131**

• **Stergios A Polyzos ET AL: "Adipocytokines and
cytokeratin-18 in patients with nonalcoholic fatty
liver disease: Introduction of CHA index
Correspondence and reprint request", , 1
September 2013 (2013-09-01), pages 749-757,
XP055391300, Retrieved from the Internet:
URL:http://www.annalsofhepatology.com/revi
sta/numeros/2013/06_135_v12n5_2013_Adipocy
tokinesCytokeratin.pdf [retrieved on 2017-07-17]**
• **FLORIANE RUDWILL ET AL: "Effect of enforced
physical inactivity induced by 60-day of bed rest
on hepatic markers of NAFLD in healthy
normal-weight women", LIVER INTERNATIONAL,
vol. 35, no. 6, 21 June 2015 (2015-06-21), pages
1700-1706, XP055391307, GB ISSN: 1478-3223,
DOI: 10.1111/liv.12743**
• **HAMZA RT1 ET AL: "Serum Pentraxin 3 Fragment
as a Noninvasive Marker of Nonalcoholic Fatty
Liver Disease in Obese Children and
Adolescents", HORMONE RESEARCH IN
PAEDIATRICS, S. KARGER AG, CH, vol. 86, no.
1, 17 June 2016 (2016-06-17), pages 11-20,
XP009504148, ISSN: 1663-2818, DOI:
10.1159/000446566**

EP 3 586 141 B1

**Description**

**FIELD OF INVENTION**

The present invention relates to the field of diagnosis in hepatology and more precisely to the diagnosis of fibrotic non-alcoholic steatohepatitis (NASH).

**BACKGROUND OF INVENTION**

Nonalcoholic steatohepatitis (NASH) is an aggressive form of nonalcoholic fatty liver disease (NAFLD). NASH has the inherent propensity to progress towards liver fibrosis which can produce progressive, irreversible liver scarring and lead to cirrhosis or towards hepatocellular carcinoma (liver cancer).

NAFLD is characterized by the presence of hepatic steatosis, *i.e.,* the accumulation of lipids within hepatocytes, said steatosis developing in the absence of significant alcohol consumption, use of steatogenic medication or hereditary disorders. In NASH, in addition to hepatic steatosis, inflammation with hepatocyte injury (ballooning) is present with or without liver fibrosis (Chalasani N et al., Gastroenterology 2012;142:1592-609).

NAFLD and NASH are generally diagnosed through the histological examination by an expert liver pathologist of a liver sample obtained by a biopsy. The NASH-CRN scoring system is commonly used by pathologists to semi-quantitatively assess the presence of steatosis, lobular inflammation, ballooning and fibrosis (Kleiner DE et al., Hepatology 2005;41:1313-21). In addition, with the NAFLD Activity Score (NAS), pathologists can allocate a score ranging from 0 to 8, corresponding to the sum of the scores for steatosis, lobular inflammation and ballooning (Brunt EM et al., Hepatology 2011;53:810-20).

In clinical practice, patients with a NAS ≥ 4 are considered to suffer from active NASH, defined by the presence of active liver injuries that are potentially reversible in the short term. Among the patients suffering from NAFLD or NASH, patients with active NASH particularly stand to benefit from therapeutic treatment since their liver injuries, while significant, can still be reversed. An additional criterion to identify the patients most susceptible to benefit from treatment is the presence of fibrosis. The presence of both an active NASH with a NAS ≥ 4 and a significant fibrosis, defined as a fibrosis stage F ≥ 2 according to the NASH-CRN staging system, characterizes patients with fibrotic NASH. Recent clinical trials, such as the REGENERATE study (NCT02548351) or the RESOLVE-IT study (NCT02704403), are conducted in patients with biopsy-proven NASH, associated to a NAS ≥ 4 and significant/advanced F2-3 fibrosis, thus illustrating the clinical relevance of identifying patients with fibrotic NASH.

While liver biopsy is still considered the gold standard to assess the presence and/or the severity of a liver condition in a subject, it does have limitations, notably due to a poor inter- or intra-observer reproducibility and a possible sample bias linked to the small size of the sample. Furthermore, liver biopsy is an invasive medical procedure and as such remains associated with a risk of complication and a significant cost. Accordingly, in recent years, non-invasive diagnostic tests have been developed to offer an alternative to liver biopsies. For example, Angulo *et al.* conceived a NAFLD Fibrosis score specifically developed for patients with NAFLD (Angulo P et al., Hepatology 2007;45:846-54). Polyzos SA *et al.* developed the CHA index (which was later mentioned in Rudwill et al., Liver Int. 2015 Jun;35(6): 1700-6), able to distinguish patients with NAFL from patients with NASH, albeit with a low sensitivity (Polyzos SA et al. Ann Hepatol. 2013 Sep-Oct;12(5):749-57). EP2490026 describes blood markers associated with NASH, such as IL-1 receptor antagonist, sCD40, HMGB1, sPLA2 group IIA, and sPLA2 activity, and methods for detecting the presence of NASH comprising measuring at least one of these markers. WO2013/049509 describes biomarkers identified as potential predictors of NASH status: ADPN, K-18 (corresponding to CK-18), C3C K-18 (corresponding to caspase 3-cleaved CK-18), IGFBP-1, ALT, and BMI. WO2013/049509 thus describes a method for identifying the presence or absence of NASH in a subject, comprising measuring at least 4 biomarkers selected from the group consisting of ADPN, K-18, C3C K-18, IGFBP-1, and ALT; and analyzing the level of the at least 4 biomarkers against reference values for those biomarkers. WO2013/049509 also describes methods for monitoring NASH in a subject or for assessing the efficacy of a therapy for treatment of NASH in a subject, said methods comprising measuring at two or more time points at least 4 biomarkers selected from the group consisting of ADPN, K-18, C3C K-18, CRP, IGFBP-1, and ALT; and analyzing the level of the at least 4 biomarkers at a second or later point in time against the values for those biomarkers at an earlier point in time. US2013/183687 teaches measuring and combining cleaved CK-18, intact CK-18, serum adiponectin and serum resistin to predict histological NASH. WO2014/049131 teaches a NASH-score comprising measuring and combining BMI, CK-18, and hyperglycemia. WO2015/192854 describes diagnosing NASH with NASH scores including sCD163, the level of alanine transaminase (ALT) and a value related to diabetes status. Hamza RT *et al.* identified pentraxin 3 (PTX3) as a biomarker that may be measured in obese children to assess the severity of fatty liver (NAFL) and/or to differentiate NASH from simple steatosis (Hamza et al., Horm Res Paediatr. 2016;86(1):11-20). However, none of the existing non-invasive methods have been developed with the aim to identify patients with fibrotic NASH.

Therefore, there remains a need for a non-invasive method to specifically and accurately assess the presence of fibrotic NASH in a subject. In particular, such a non-invasive test would represent a convenient and practical manner to assess the presence of fibrotic NASH in a subject, to monitor patients suffering from NAFLD or NASH for the development of fibrotic NASH and to monitor treatment response in a subject treated for fibrotic NASH.

The present invention thus relates to a method for assessing the presence and/or the severity of fibrotic NASH, in a subject comprising measuring cytokeratin 18 (CK-18), aspartate aminotransferase (AST) and Homeostasis model assessment of insulin resistance (HOMA) in a sample of said subject, combining the three obtained measures in a binary logistic regression to obtain a score value ranging from 0 to 1, and comparing the score value to at least one predetermined reference value, wherein said predetermined reference value is derived from the determination of the score in one or more subjects who are identified as suffering from NAFLD, NASH, or fibrotic NASH. The present invention also relates to monitoring methods comprising measuring CK-18, AST and HOMA in a sample of the subject and combining the three obtained measures in a binary logistic regression in order to determine a score value ranging from 0 to 1. The monitoring methods of the invention also comprise either comparing the score value to a personalized reference value for monitoring the progression of fibrotic NASH in a subject, or comparing score values obtained before and after the beginning of a treatment for monitoring treatment response in a subject treated for fibrotic NASH.

**SUMMARY**

The present invention relates to a method for assessing the presence and/or the severity of fibrotic NASH in a subject, wherein said method comprises:

- measuring cytokeratin 18 (CK-18), aspartate aminotransferase (AST) and Homeostasis model assessment of insulin resistance (HOMA) in a sample of said subj ect;
- combining the three obtained measures in a binary logistic regression to obtain a score value ranging from 0 to 1; and
- comparing the score value to at least one predetermined reference value, wherein said predetermined reference value is derived from the determination of the score in one or more subjects who are identified as suffering from non-alcoholic fatty liver disease (NAFLD), NASH, or fibrotic NASH,

thereby assessing the presence and/or the severity of fibrotic NASH in the subject.

In one embodiment, the method of the invention is computerized.

In one embodiment, the score value is compared to two predetermined reference values. In one embodiment, the first predetermined reference value is an exclusion cut-off, preferably ranging from about 0.1 to about 0.2. In one embodiment, the second predetermined reference value is an affirmation cut-off, preferably ranging from about 0.5 to about 0.6.

In one embodiment, the sample of the subject is a serum sample, a plasma sample or a blood sample.

In one embodiment, the subject is an animal, preferably a human. In one embodiment, the subject has a preexisting disease or condition, preferably metabolic syndrome and/or non-alcoholic fatty liver disease (NAFLD).

In one embodiment, the method of the invention is performed for a subject who has been previously diagnosed with non-alcoholic fatty liver disease (NAFLD), and comprises the additional step of first assessing the presence of metabolic syndrome in the subject suffering from NAFLD and measuring the level of aspartate aminotransferase (AST) in a sample of said subject suffering from NAFLD, before carrying out the steps as described hereinabove.

The present invention also relates to a method for monitoring the progression of fibrotic NASH in a subject, wherein said method comprises:

a) determining a score value, said score value ranging from 0 to 1, by measuring in a sample of said subject CK-18, AST and Homeostasis model assessment of insulin resistance (HOMA) and combining the three obtained measures in a binary logistic regression;
b) comparing the score to a personalized reference value.

In one embodiment, the personalized reference value for a subject is a score value previously determined for said subject.

In one embodiment, a score value is determined at least twice over a period of time.

The present invention also relates to a method for monitoring treatment response in a subject treated for fibrotic NASH, wherein said method comprises:

- determining a score, said score value ranging from 0 to 1, before the beginning of the treatment by measuring in a sample of said subject cytokeratin 18 (CK-18), aspartate aminotransferase (AST) and Homeostasis model assessment of insulin resistance (HOMA), and combining the three obtained measures in a binary logistic regression;
- repeating the determination of the score value after the beginning of the treatment; and

- comparing the scores values obtained before and after the beginning of the treatment, thereby determining whether said treatment is effective.

In one embodiment, the determination of the score value after the beginning of the treatment is repeated at least twice, and preferably at regular intervals.

DEFINITIONS

In the present invention, the following terms have the following meanings:

- The terms **"a"** and **"an"** refer to one or to more than one (*i.e.,* to at least one) of the grammatical object of the article. By way of example, "an element" means one element or more than one element.

- **"About",** preceding a figure means plus or minus 10% of the value of said figure.

- **"Accuracy"** of a diagnostic test refers to the proportion of correctly diagnosed patients, *i.e.,* the proportion of patients with correctly identified fibrotic NASH by said diagnostic test.

- **"AUROC"** stands for area under the ROC curve, and is an indicator of the accuracy of a diagnostic test. In statistics, a receiver operating characteristic (ROC), or ROC curve, is a graphical plot that illustrates the performance of a binary classifier system as its discrimination threshold is varied. The curve is created by plotting the sensitivity against the specificity (usually 1- specificity) at successive values from 0 to 1. ROC curve and AUROC are well-known in the field of statistics.

- **"Biomarker"** or **"biological marker"** refers to a variable that may be measured in a sample from the subject, said sample being a bodily fluid sample, such as, for example, a blood, plasma, serum, lymph, urine, cerebrospinal fluid or sweat sample, preferably a blood, plasma, serum or urine sample, more preferably a blood, plasma or serum sample.

- **"Diagnostic target"** refers to the main objective of a non-invasive diagnostic test. For example, a non-invasive diagnostic test wherein the diagnostic target is fibrotic NASH aims at assessing the presence and/or the severity of fibrotic NASH in a subj ect.

- **"Fibrosis",** in the present invention, specifically refers to liver fibrosis, a pathological lesion of the liver made of scar tissue including fibrillary proteins or glycoproteins (collagens, proteoglycans...).

- **"Fibrotic NASH",** refers to an active NASH characterized by a NAFLD Activity Score (NAS) $\geq$ 4 and the presence of significant fibrosis, defined as a fibrotic stage F $\geq$ 2, preferably according to the NASH-CRN staging system.

- **"NAFLD Activity Score (NAS)":** refers to a system of scoring the histological features of nonalcoholic fatty liver disease (NAFLD). The NAS ranges from 0 to 8, and corresponds to the sum of the scores for steatosis, lobular inflammation and ballooning. The NAS scoring system is commonly used for the histological diagnosis of NASH, defined as the presence of a score $\geq$ 1 for each of the three components of the NAS. In one embodiment, a NAS score $\geq$ 4 defines an active NASH.

- **"NAFLD lesions",** in the present invention, encompasses all the lesions, or diseases, that correspond to the definition of nonalcoholic fatty liver disease (NAFLD) and are characterized by the presence of hepatic steatosis, *i.e.,* the accumulation of lipids within hepatocytes. Thus, according to the present invention, NAFLD lesions include NAFLD, NASH, active NASH, fibrotic NASH, cirrhotic NASH, NAFLD with advanced fibrosis (*i.e.,* NAFLD with advanced fibrosis and without NASH and/or without active NASH), NAFLD with cirrhosis (NAFLD with cirrhosis and without NASH and/or without active NASH).

- **"NASH-CRN scoring system"** (NASH Clinical Research Network scoring system) refers to a classification system devoted to NAFLD (non-alcoholic fatty liver disease) and based on a morphological description in different classes either for steatosis (conventionally referred as grading) or fibrosis (conventionally referred as staging). This semi-quantitative (ordinal in statistics) system is the most recent and conventional histological classification. This system is also known as the Kleiner grading/staging system.

- **"NASH"** (nonalcoholic steatohepatitis) refers to the presence of a score $\geq 1$ for each of the three components of the NAS: steatosis, lobular inflammation and ballooning.

- **"Non-invasive"** when referring to a method or test according to the present invention, means that the method or test of the invention does not comprise obtaining a tissue sample from the body of a subject. In one embodiment, a blood sample is not considered as a tissue sample.

- **"Prevalence"** is the proportion of patients, or rate of patients, found to be affected by the diagnostic target, *i.e.,* the disease tested, in the population of patients considered. According to the present invention, the prevalence is expressed as a percentage. In one embodiment, the prevalence of fibrotic NASH corresponds to the proportion of patients, or rate of patients, found to have fibrotic NASH in the population of patients considered.

- **"Score",** as in score value, refers to any digit value obtained by the mathematical combination of biomarkers. According to the present invention, a score is a bound digit value, obtained by a binary logistic regression. According to the present invention, the score ranges from 0 to 1.

- **"Sensitivity",** for a non-invasive test, measures the proportion of patients with the diagnostic target that are correctly identified as such (*i.e.,* the percentage of patients with the diagnostic target who are correctly identified by a positive non-invasive test).

- **"Specificity",** for a non-invasive test, measures the proportion of patients without the diagnostic target that are correctly identified as such (*i.e.,* the percentage of patients without the diagnostic target who are correctly identified by a negative non-invasive test).

- **"Subject"** refers to a mammal, preferably a human.

- **"Treating" or "treatment"** refers to both therapeutic treatment and prophylactic or preventative measures, wherein the object is to prevent or slow down (lessen) the development of fibrotic NASH. Those in need of treatment include those already with fibrotic NASH, as well as those susceptible to develop fibrotic NASH, or those in whom fibrotic NASH is to be prevented. A subject is successfully "treated" for fibrotic NASH if, after receiving a therapeutic amount of a therapeutic agent, the patient shows one or more of the following:

  - Significant improvement of liver lesions defined as:

    - Decrease in fibrosis stage;
    - Improvement in NAS by at least 2 points with no worsening of fibrosis;
    - Reversal of NASH without worsening of fibrosis, said reversal of NASH corresponding to the absence (score of 0) of at least one of the 3 components of the NAS (*i.e.,* steatosis, ballooning, and inflammation); and/or
    - Resolution of NASH without worsening of liver fibrosis, said resolution of NASH corresponding to the disappearance of ballooning (score = 0) together with either the disappearance of lobular inflammation or the persistence of mild lobular inflammation only (score = 0 or 1);

  - Relief to some extent, of one or more of the symptoms associated with fibrotic NASH;
  - Reduced morbidity and mortality; and/or
  - Improvement in quality of life issues.

The above parameters for assessing successful treatment and improvement in fibrotic NASH are readily measurable by routine procedures familiar to a physician.

**DETAILED DESCRIPTION**

The present invention relates to a method for assessing the presence and/or the severity of fibrotic NASH, in a subject comprising measuring cytokeratin 18 (CK-18), aspartate aminotransferase (AST) and Homeostasis model assessment of insulin resistance (HOMA) in a sample of said subject; combining the three obtained measures in a binary logistic regression to obtain a score value ranging from 0 to 1; and comparing the score value to at least one predetermined reference value, wherein said predetermined reference value is derived from the determination of the score in one or more subjects who are identified as suffering from NAFLD, NASH, or fibrotic NASH, thereby assessing the presence and/or the severity of fibrotic NASH in the subject.

Indeed, the Applicant has surprisingly shown that measuring CK-18, AST and HOMA, and combining the three obtained measures in a binary logistic regression to obtain a score value ranging from 0 to 1, allows to accurately assess the presence of NASH, especially fibrotic NASH, in a subject. As demonstrated through the comparison of their diagnostic performance, for example with the evaluation of their respective AUROCs or of their overall diagnostic accuracy, the method of the invention is more accurate, both for the diagnosis of NASH and fibrotic NASH, than the tests of the prior art, such as, for example, more accurate than the NAFLD Fibrosis score (Angulo P et al., Hepatology 2007;45:846-54) and more accurate than the NASH-score of WO2014/049131.

In particular, regarding the diagnosis of fibrotic NASH, the Applicant has surprisingly shown that the method of the invention is more accurate than several combinations of markers described in WO2014/049131 and than the CHA index as described in Polyzos SA et al. Ann Hepatol. 2013 Sep-Oct;12(5):749-57.

In one embodiment, the method of the invention is a non-invasive method. In one embodiment, the method of the invention is an *in vitro* diagnosis method.

Nonalcoholic Fatty Liver Disease (NAFLD) is characterized by the presence of hepatic steatosis, said steatosis developing in the absence of significant alcohol consumption, use of steatogenic medication or hereditary disorders.

NAFLD encompasses nonalcoholic steatohepatitis (NASH), which is defined as the presence of hepatic steatosis concomitantly with inflammation and hepatocyte injury (ballooning), with or without fibrosis (definition of the AASLD Practice Guideline, Chalasani et al., Hepatology, 55(6):2005-2023, 2012).

Among patients with NASH, patients with a NAFLD Activity Score lower than 3 (NAS < 3) are considered to suffer from inactive NASH. By contrast, patients with a NAFLD Activity Score greater than or equal to 4 (NAS $\geq$ 4) are considered to suffer from active NASH, defined by the presence of active liver injuries that are potentially reversible in the short term.

According to the present invention, patients suffering from active NASH, with a NAFLD Activity Score greater than or equal to 4 (NAS $\geq$ 4), concomitantly with significant fibrosis, defined as a fibrosis stage greater than or equal to 2 (F $\geq$ 2, preferably according to the NASH CRN staging system), are considered to suffer from fibrotic NASH.

In one embodiment, the method of the invention is for assessing the presence and/or the severity of active NASH in a subject. In another embodiment, the method of the invention is for assessing the presence and/or the severity of inactive NASH in a subject.

In one embodiment, the method of the invention is for assessing the presence and/or the severity of fibrotic NASH, in a subject, wherein said subject has been previously diagnosed with NAFLD.

In one embodiment which is outside the subject-matter of the claims, the method consists in measuring cytokeratin 18 (CK-18), aspartate aminotransferase (AST) and Homeostasis model assessment of insulin resistance (HOMA) in a sample of said subject.

In one embodiment, the method of the invention does not comprise measuring any additional biomarkers. In one embodiment, the method of the invention does not comprise measuring BMI and/or hyperglycemia.

In one embodiment, the method of the invention comprises:

- measuring cytokeratin 18 (CK-18), aspartate aminotransferase (AST) and Homeostasis model assessment of insulin resistance (HOMA) in a sample of the subject; and
- determining the presence of fibrotic NASH, based on the measures of CK-18, AST and HOMA.

In another embodiment which is outside the subject-matter of the claims, the method comprises:

- measuring CK-18, AST and HOMA in a sample of the subject;
- comparing the measures obtained for CK-18, AST and HOMA to predetermined values of CK-18, AST and HOMA.

In another embodiment which is outside the subject-matter of the claims, the method comprises:

- measuring CK-18, AST and HOMA in a sample of the subject;
- comparing the measures obtained for CK-18, AST and HOMA to predetermined values of CK-18, AST and HOMA;

thereby determining the presence of NASH, preferably fibrotic NASH, in the subject.

As used herein, the term "predetermined values of CK-18, AST and HOMA" broadly encompasses any suitable reference values which may be used as a basis for comparison with respect to the measured CK-18, AST and HOMA.

Predetermined values of CK-18, AST and HOMA can be relative to a number or value derived from population studies, including without limitation, such subjects having similar age range, subjects in the same or similar ethnic group, subjects having family histories of liver condition or disease, or subjects having family histories of NAFLD.

In one embodiment which is outside the subject-matter of the claims, the predetermined values of CK-18, AST and HOMA are derived from the measure of CK-18, AST and HOMA, respectively, in one or more subjects who are substantially healthy. As used herein, a "substantially healthy subject" has not been previously diagnosed or identified as having or suffering from a liver condition or disease.

In another embodiment which is outside the subject-matter of the claims, the predetermined values of CK-18, AST and HOMA are derived from the measure of CK-18, AST and HOMA, respectively, in one or more subjects who are diagnosed or identified as having or suffering from NAFLD, NASH, or fibrotic NASH.

Another object which is outside the subject-matter of the claims is a method for assessing CK-18, AST and HOMA in a subject, said method comprising:

- measuring CK-18, AST and HOMA in a sample of the subject;
- comparing the measures obtained for CK-18, AST and HOMA to predetermined values of CK-18, AST and HOMA.

Caspase-cleaved cytokeratin 18 (CK-18) is a marker of apoptosis. In particular, CK-18 is released from hepatocytes during apoptosis and can thus be measured in a sample obtained from the subject, such as in a blood, plasma or serum sample.

Methods for measuring CK-18 include immunoassay techniques involving the use of one or more monoclonal or polyclonal antibodies. Immunoassays techniques include radioimmunoassay or RIA, ELISA assays and western blot. Antibodies recognizing CK-18 are commercially available, such as for example, from Santa Cruz Biotech or Abcam.

In one embodiment, the serum level of apoptotic caspase-3 generated CK-18 fragments is measured with the M30-Apoptosense enzyme-linked immunosorbent assay kit (PEVIVA, Bromma, Sweden).

In one embodiment, the level of CK-18 is expressed in IU/L.

Aspartate aminotransferase (AST or ASAT) is an enzyme mainly present in the muscles, heart and liver. Elevated AST activity may be a marker of liver inflammation and is commonly measured in a sample obtained from the subject for diagnosing a liver impairment or disease.

Methods for measuring the AST activity in a sample obtained from the subject are well-known. Examples of such methods include colorimetric analysis, spectrophotometric measurement, chemiluminescence, chromatography, fluorescence and UV absorbance, radiochemical analysis, or electrochemical techniques.

In one embodiment, AST activity is measured with an analyzer, such as a Roche/Hitachi 917 (Roche Diagnostics) or a Beckman Coulter UniCel® DxC800 Synchron Clinical.

In one embodiment, the level of AST is expressed in IU/L.

HOMA or Homeostasis model assessment of insulin resistance is a test assessing β-cell function and insulin resistance (IR) from fasting glucose and insulin or C-peptide concentrations, first described in 1985 (Matthews et al., Diabetologia 28:412-419, 1985). In the present invention, HOMA may also be referred to as HOMA-IR (Homeostasis model assessment of insulin resistance).

In one embodiment, HOMA is assessed in a sample obtained from the subject using the following formula:

$$\text{HOMA-IR} = [\text{glucose (mmol/L)} * \text{insulin (}\mu\text{U/mL)}/22.5], \text{ using fasting values.}$$

In one embodiment, HOMA is assessed in a sample obtained from the subject using the logarithmic transformation of HOMA.

In one embodiment, the HOMA value is capped. In one embodiment, the HOMA is capped at a value ranging from about 5 to about 20, preferably at a value ranging from about 7 to about 15, and more preferably is capped at about 10.

Without wishing to be bound to any theory, the Applicant proposes that capping the HOMA value limits the influence of supra-physiological results of insulinemia, notably induced by insulin therapy, on the score of the invention described below.

According to the present invention, HOMA is a biomarker reflecting the glucose metabolism of the subject in whom HOMA is measured. In one embodiment which is outside the subject-matter of the claims, alternative biomarkers reflecting the glucose metabolism of the subject may be used. Examples of such alternative markers include fasting glucose-to-insulin ratio and QUICKI.

QUICKI or quantitative insulin sensitivity check index is a method for assessing insulin sensitivity (Katz A et al., J Clin Endocrinol Metab 85: 2402-2410, 2000). QUICKI is derived from logarithmic-transformed fasting plasma glucose (FPG) and insulin levels.

QUICKI is assessed in a sample obtained from the subject using the following formula:

$$\text{QUICKI} = 1 / [\log(\text{Fasting Insulin}) + \log(\text{Fasting Glucose})].$$

In one embodiment which is outside the subject-matter of the claims, the measure of HOMA is replaced by the measure of the alternative biomarker fasting glucose-to-insulin ratio. Therefore, the present disclosure also relates to a method for assessing the presence and/or the severity of NASH, preferably fibrotic NASH, in a subject comprising measuring CK-18, AST and fasting glucose-to-insulin ratio in a sample of said subject, said method being outside the subject-matter of the claims.

In another embodiment which is outside the subject-matter of the claims, the measure of HOMA is replaced by the measure of the alternative biomarker QUICKI. Therefore, the present disclosure also relates to a method for assessing the presence and/or the severity of NASH, preferably fibrotic NASH, in a subject comprising measuring CK-18, AST and QUICKI in a sample of said subject, said method being outside the subject-matter of the claims.

Thus, in one embodiment which is outside the subject-matter of the claims, the method for assessing the presence and/or the severity of NASH, preferably fibrotic NASH, in a subject comprising measuring cytokeratin 18 (CK-18), aspartate aminotransferase (AST) and one of Homeostasis model assessment of insulin resistance (HOMA), fasting glucose-to-insulin ratio or QUICKI in a sample of said subject.

The method of the invention does not comprise obtaining a sample from a subject. The sample of the subject is a sample previously obtained from the subject. Said sample may be conserved in adequate conditions before being used in the method of the invention.

In one embodiment, the sample obtained from the subject is a body fluid sample. Examples of body fluids include blood, plasma, serum, lymph, urine, cerebrospinal fluid or sweat.

In one embodiment, the sample obtained from the subject is a blood sample. In one embodiment, the sample obtained from the subject is a whole blood sample or a plasma sample. In one embodiment, the whole blood sample or the plasma sample obtained from the subject is processed to obtain a serum sample. Methods for obtaining a serum sample from a whole blood sample or a plasma sample are routinely used in clinical laboratories.

In one embodiment which is outside the subject-matter of the claims, the method further comprises combining the measures of CK-18, AST and HOMA in a mathematical function to obtain a score value (or alternatively combining the measures of CK-18, AST and fasting glucose-to-insulin ratio in a mathematical function to obtain a score value, or alternatively combining the measures of CK-18, AST and QUICKI in a mathematical function to obtain a score value).

Thus, in one embodiment which is not encompassed by the subject-matter of the claims, the disclosure relates to a method for assessing the presence and/or the severity of NASH, preferably fibrotic NASH, in a subject comprising:

- measuring CK-18, AST and HOMA in a sample of said subject; and
- combining the measures of CK-18, AST and HOMA in a mathematical function to obtain a score value.

In one embodiment which is outside the subject-matter of the claims, the method consists in:

- measuring CK-18, AST and HOMA in a sample of said subject; and
- combining the measures of CK-18, AST and HOMA in a mathematical function to obtain a score value.

In one embodiment which is outside the subject-matter of the claims, the method comprises:

- measuring cytokeratin 18 (CK-18), aspartate aminotransferase (AST) and one of Homeostasis model assessment of insulin resistance (HOMA), fasting glucose-to-insulin ratio or QUICKI in a sample of said subject; and
- combining the three obtained measures in a mathematical function to obtain a score value.

In another embodiment which is not encompassed by the subject-matter of the claims, the method comprises:

- measuring CK-18, AST and HOMA in a sample of said subject; and
- combining the measures of CK-18, AST and HOMA in a mathematical function to obtain a score value;

thereby assessing the presence in the subject of NASH, preferably fibrotic NASH, based on said score value.

In one embodiment which is not encompassed by the subject-matter of the claims, the method comprises comparing the score value to at least one predetermined reference value.

As used herein, the term "reference value" broadly encompasses any suitable reference value which may be used as a basis for comparison with respect to the obtained score.

A reference value can be relative to a number or value derived from population studies, including without limitation, such subjects having similar age range, subjects in the same or similar ethnic group, subjects having family histories of liver condition or disease, or subjects having family histories of NAFLD.

In one embodiment, the reference value is constructed using algorithms and other methods of statistical and structural classification.

In one embodiment which is outside the subject-matter of the claims, the reference value is derived from the determination of the score in one or more subjects who are substantially healthy. As used herein, a "substantially healthy subject" has not been previously diagnosed or identified as having or suffering from a liver condition or disease.

According to the present invention, the reference value is derived from the determination of the score in one or more subjects who are diagnosed or identified as having or suffering from NAFLD, NASH, or fibrotic NASH.

In one embodiment, the method of the invention comprises comparing the score value to a reference value, wherein:

- a score value inferior to the reference value indicates that fibrotic NASH is not present in the subject;
- a score value superior to the reference value indicates that fibrotic NASH is present in the subject.

In one embodiment, the method of the invention comprises comparing the score value to two predetermined reference values.

In one embodiment, the first predetermined reference value corresponds to the exclusion cut-off and a score value below said exclusion cut-off is indicative of the absence of fibrotic NASH. In one embodiment, the second predetermined reference value corresponds to the affirmation cut-off and a score value above said affirmation cut-off is indicative of the presence of fibrotic NASH. In one embodiment, the interval between the two predetermined reference values, preferably between the exclusion cut-off and the affirmation cut-off, corresponds to an indeterminate zone. In one embodiment, a score value within the indeterminate zone indicates that no conclusion may be drawn regarding the presence or absence of fibrotic NASH, and additional tests may be required.

In one embodiment, the method of the invention comprises comparing the score value to two predetermined reference values, preferably an exclusion cut-off and an affirmation cut-off, corresponding to an 80, 85, 90 or 95% sensitivity and an 80, 85, 90 or 95% specificity. In one embodiment, the method of invention comprises comparing the score value to two predetermined reference values, preferably an exclusion cut-off and an affirmation cut-off, corresponding to a 90% sensitivity and a 95% specificity.

In one embodiment, the score value ranges from 0 to 1; the first predetermined reference value, preferably an exclusion cut-off, ranges from about 0.05 to about 0.25, preferably from about 0.1 to about 0.2 and more preferably from about 0.12 to about 0.15, such as, for example, about 0.133 or about 0.134; and the second predetermined reference value, preferably an affirmation cut-off, ranges from about 0.45 to about 0.65, preferably from about 0.5 to about 0.6 and more preferably from about 0.52 to about 0.55 or from about 0.53 to about 0.56, such as, for example, about 0.533 or about 0.550.

In one embodiment, the method of the invention is for assessing the presence of fibrotic NASH in a subject and the method comprises comparing the score value to two predetermined reference values, preferably an exclusion cut-off and an affirmation cut-off, wherein:

- a score value equal or inferior to the first predetermined reference value, preferably an exclusion cut-off, indicates that fibrotic NASH is not present in the subject;
- a score value equal or superior to the second predetermined reference value, preferably an affirmation cut-off, indicates that fibrotic NASH is present in the subject; and
- a score value between the first and the second predetermined reference value, preferably between an exclusion cut-off and an affirmation cut-off, indicates that further testing is required to assess whether fibrotic NASH is present in the subject.

In one embodiment, the method of the invention is for assessing the presence of fibrotic NASH in a subject and the method comprises comparing the score value to two predetermined reference values, preferably an exclusion cut-off and an affirmation cut-off, corresponding to a 90% sensitivity and a 95% specificity, wherein the score value ranges from 0 to 1, the first predetermined reference value, preferably an exclusion cut-off, ranges from about 0.05 to about 0.25 and the second predetermined reference value, preferably an affirmation cut-off, ranges from about 0.45 to about 0.65, and wherein:

- a score value equal or inferior to the first predetermined reference value, preferably an exclusion cut-off, indicates that fibrotic NASH is not present in the subject;
- a score value equal or superior to the second predetermined reference value, preferably an affirmation cut-off, indicates that fibrotic NASH is present in the subject; and
- a score value between the first and the second predetermined reference values, preferably between an exclusion cut-off and an affirmation cut-off, indicates that further testing is required to assess whether fibrotic NASH is present in the subject.

In one embodiment which is not encompassed by the subject-matter of the claims, the mathematical function is a binary logistic regression, a multiple linear regression or any multivariate analysis, preferably a binary logistic regression.

In one embodiment which is not encompassed by the subject-matter of the claims, the score value is obtained by combining the measures of CK-18, AST and HOMA in a regression formula established using multivariate analysis. According to the present invention, said regression is a binary logistic regression.

In one embodiment, said formula is expressed as:

A + B1 * (CK-18) + B2 * (AST) + B3 * (HOMA), wherein A, B1, B2 and B3 are predetermined coefficients.

In another embodiment, said formula is expressed as:

C + [D1 * LN(CK-18)] + [D2 * LN(AST)] + [D3 * LN(HOMA)], wherein C, D1, D2 and D3 are predetermined coefficients.

Another object which is outside the subject-matter of the claims is a score, obtained by measuring CK-18, AST and HOMA in a sample of a subject and combining the measures of CK-18, AST and HOMA in a mathematical function. Alternatively, the score is obtained by measuring CK-18, AST and fasting glucose-to-insulin ratio in a sample of a subject and combining the measures of CK-18, AST and fasting glucose-to-insulin ratio in a mathematical function. In another alternative which is outside the subject-matter of the claims, the score is obtained by measuring CK-18, AST and QUICKI in a sample of a subject and combining the measures of CK-18, AST and QUICKI in a mathematical function.

In one embodiment which is outside the subject-matter of the claims, the score is obtained by measuring cytokeratin 18 (CK-18), aspartate aminotransferase (AST) and one of Homeostasis model assessment of insulin resistance (HOMA), fasting glucose-to-insulin ratio or QUICKI in a sample of a subject and combining the three obtained measures in a mathematical function.

In one embodiment, the subject is a male. In another embodiment, the subject is a female.

In one embodiment, the subject is an adult. According to the present invention, an adult is a subject above the age of 18, 19, 20 or 21 years. In another embodiment, the subject is a child. According to the present invention, a child is a subject below 21, 20, 19 or 18 years, such as, for example, 17, 16, 15, 14, 13, 12, 11, 10 or 9 years.

In one embodiment, the subject is a patient, *i.e.*, a warm-blooded animal, more preferably a human, who/which is awaiting the receipt of, or is receiving, medical care or was/is/will be the subject of a medical procedure, or is monitored for the development or progression of a disease. In one embodiment, the subject is a human patient who is treated and/or monitored for the development or progression of a liver disease or condition (preferably NAFLD, NASH or fibrotic NASH).

In one embodiment, the subject has a preexisting liver disease or condition, preferably NAFLD. In one embodiment, the subject has a preexisting disease or condition, preferably metabolic syndrome. Examples of metabolic syndrome include diabetes, hypertension, dyslipidemia, abetalipoproteinemia, glycogen storage diseases, Weber-Christian disease, Wolman disease, and lipodystrophy. In one embodiment, the subject has a preexisting NAFLD and/or a metabolic syndrome.

In one embodiment, the subject is diabetic. In another embodiment, the subject is not diabetic.

In one embodiment, the method of the invention comprises the additional step of first assessing the presence of metabolic syndrome and measuring the level of aspartate aminotransferase (AST) in a sample of the subject before carrying out the steps as described hereinabove.

In one embodiment, the method of the invention is performed for a subject who has been previously diagnosed with non-alcoholic fatty liver disease (NAFLD), and comprises the additional step of first assessing the presence of metabolic syndrome and measuring the level of aspartate aminotransferase (AST) in a sample of said subject suffering from NAFLD, before carrying out the steps as described hereinabove.

Thus, another object of the present invention is a method for assessing the presence and/or the severity of fibrotic NASH in a subject, wherein said method comprises the steps of:

- first assessing the presence of metabolic syndrome in the subject and measuring the level of aspartate aminotransferase (AST) in a sample of said subj ect;
- then assessing the presence and/or the severity of fibrotic NASH as described hereinabove.

In one embodiment, the method of the invention is a method for assessing the presence and/or the severity of fibrotic NASH in a patient diagnosed with non-alcoholic fatty liver disease (NAFLD), wherein said method comprises the steps of:

- first assessing the presence of metabolic syndrome in a NAFLD patient and measuring the level of aspartate aminotransferase (AST) in a sample of said NAFLD patient;
- then assessing the presence and/or the severity of fibrotic NASH as described hereinabove.

In one embodiment, the level of aspartate aminotransferase is compared to a predetermined value.

As used herein, the term "predetermined value" broadly encompasses any suitable reference value which may be used as a basis for comparison with respect to the measured level of AST.

A predetermined value can be relative to a number or value derived from population studies, including without limitation, such subjects having similar age range, subjects in the same or similar ethnic group, subjects having family histories of liver condition or disease, or subjects having family histories of NAFLD.

In one embodiment, the predetermined value is derived from the measure of AST level in one or more subjects who are substantially healthy. As used herein, a "substantially healthy subject" has not been previously diagnosed or identified as having or suffering from a liver condition or disease.

In another embodiment, the predetermined value is derived from the measure of AST level in one or more subjects who are diagnosed or identified as having or suffering from NAFLD, NASH, or fibrotic NASH.

In one embodiment, the predetermined value of AST ranges from about 20 UI/L to about 50 UI/L, preferably from about 25 UI/L to about 45 UI/L and more preferably from about 30 UI/L to about 40 UI/L. In one embodiment, the predetermined value of AST is about 32, 33, 34, 35, 36, 37, or 38 UI/L, more preferably about 35 UI/L.

In one embodiment, carrying the method of the invention to assess the presence and/or the severity of fibrotic NASH is not required for subjects, preferably NAFLD patients, who do not have metabolic syndrome and who have a measured AST level lower than a predetermined value, preferably lower than about 32, 33, 34, 35, 36, 37, or 38 UI/L, more preferably lower than about 35 UI/L. As demonstrated in Example 1, the presence of fibrotic NASH can be excluded in patients suffering from NAFLD who do not have metabolic syndrome and who have a measured AST level lower than a predetermined value, preferably lower than about 32, 33, 34, 35, 36, 37, or 38 UI/L, more preferably lower than about 35 UI/L.

In one embodiment, carrying the method of the invention to assess the presence and/or the severity of fibrotic NASH is required for subjects, preferably NAFLD patient, who also have metabolic syndrome and/or a measured AST level greater than or equal to a predetermined value, preferably greater than or equal to about 32, 33, 34, 35, 36, 37, or 38 UI/L, more preferably greater than or equal to about 35 UI/L. As demonstrated in Example 1, the presence and/or severity of fibrotic NASH should preferably be assessed by the method of the invention in patients suffering from NAFLD who also have metabolic syndrome, in patients suffering from NAFLD who also have a measured AST level greater than or equal to a predetermined value, preferably greater than or equal to about 32, 33, 34, 35, 36, 37, or 38 UI/L, more preferably greater than or equal to about 35 UI/L, and in patients suffering from NAFLD who have both a metabolic syndrome and a measured AST level greater than or equal to a predetermined value, preferably greater than or equal to about 32, 33, 34, 35, 36, 37, or 38 UI/L, more preferably greater than or equal to about 35 UI/L.

Thus, in one embodiment, the method of the invention for assessing the presence and/or the severity of fibrotic NASH in a patient diagnosed with non-alcoholic fatty liver disease (NAFLD), comprises:

- assessing the presence of metabolic syndrome in a NAFLD patient; and
- measuring the level of aspartate aminotransferase (AST) in a sample of said NAFLD patient and comparing the measured AST level to a predetermined value.

According to a first embodiment which is outside the subject-matter of the claims, if

- the NAFLD patient does not have a metabolic syndrome; and
- the measured level of AST is lower than a predetermined value, for example lower than about 32, 33, 34, 35, 36, 37, or 38 UI/L, in particular lower than about 35 UI/L;

then the presence of NASH, preferably fibrotic NASH, is excluded.

According to a second embodiment, if

- the NAFLD patient has a metabolic syndrome; or
- the measured level of AST is greater than or equal to a predetermined value, for example greater than or equal to about 32, 33, 34, 35, 36, 37, or 38 UI/L, in particular greater than or equal to 35 UI/L; or
- the NAFLD patient has a metabolic syndrome and a measured level of AST greater than or equal to a predetermined value, for example greater than or equal to about 32, 33, 34, 35, 36, 37, or 38 UI/L, in particular greater than or equal to about 35 UI/L;

then the presence of fibrotic NASH is to be assessed by the method of the invention as described hereinabove.

Accordingly, in one embodiment which is not encompassed by the subject-matter of the claims, the method for assessing the presence and/or the severity of NASH, preferably fibrotic NASH, in a patient diagnosed with non-alcoholic fatty liver disease (NAFLD), comprises:

- in a first step, assessing the presence of metabolic syndrome in a NAFLD patient and measuring the level of aspartate aminotransferase (AST) in a sample of said NAFLD patient and comparing the measured level to a predetermined value, for example about 32, 33, 34, 35, 36, 37, or 38 UI/L, in particular about 35 UI/L; and if the NAFLD patient has a metabolic syndrome, or a measured level of AST greater than or equal to a predetermined value; or both

- in a second step, assessing the presence and/or the severity of NASH, preferably fibrotic NASH, by measuring cytokeratin 18 (CK-18), aspartate aminotransferase (AST) and Homeostasis model assessment of insulin resistance (HOMA) in a sample of said subject and optionally combining the measures of CK-18, AST and HOMA in a mathematical function to obtain a score value as described hereinabove.

In one embodiment of the invention, the method is computerized. Thus, in one embodiment, the invention relates to a computer-implemented method for assessing the presence and/or the severity of fibrotic NASH in a subject.

In one embodiment, the invention relates to a computer-implemented method for assessing the presence and/or the severity of fibrotic NASH in a subject comprising:

- measuring CK-18, AST and HOMA in a sample of said subject; and
- combining the measures of CK-18, AST and HOMA in a mathematical function to obtain a score value as described hereinabove.

Another object which is outside the subject-matter of the claims is thus a computer software for implementing the method of the invention.

In one embodiment, the method of the invention is implemented with a microprocessor comprising a software configured to calculate a score value resulting from the combination of the measures of CK-18, AST and HOMA.

The present disclosure also relates to a diagnostic test for implementing the method as described hereinabove, said diagnostic test being outside the subject-matter of the claims.

Thus, one object which is outside the subject-matter of the claims is a diagnostic test comprising measuring CK-18, AST and HOMA in a sample of said subject.

In one embodiment which is outside the subject-matter of the claims, the diagnostic test comprises measuring CK-18, AST and HOMA in a sample of said subject and comparing the measures obtained to predetermined values of CK-18, AST and HOMA as described hereinabove. In another embodiment which is outside the subject-matter of the claims, the diagnostic test comprises measuring CK-18, AST and HOMA in a sample of said subject and combining the measures of CK-18, AST and HOMA in a mathematical function to obtain a score value as described hereinabove. In yet another embodiment which is outside the subject-matter of the claims, the diagnostic test comprises measuring CK-18, AST and HOMA in a sample of said subject, combining the measures of CK-18, AST and HOMA in a mathematical function to obtain a score value and comparing the score value to a reference value, as described hereinabove.

The present disclosure also relates to a kit for implementing the method as described hereinabove, said kit being outside the subject-matter of the claims. According to the disclosure, the kit, which is outside the subject-matter of the claims, comprises means for measuring CK-18, AST and HOMA in a sample obtained, preferably previously obtained, from a subject.

The present disclosure also relates to a method for monitoring the progression of NASH, preferably fibrotic NASH, in a subject, comprising measuring cytokeratin 18 (CK-18), aspartate aminotransferase (AST) and Homeostasis model assessment of insulin resistance (HOMA) in a sample of the subject at least twice, and preferably at regular intervals, said method being outside the subject-matter of the claims.

In one embodiment which is outside the subject-matter of the claims, said method comprises:

- measuring CK-18, AST and HOMA in a sample of the subject, optionally comparing the measures obtained for CK-18, AST and HOMA to predetermined values of CK-18, AST and HOMA;
- repeating the measures at least twice, and preferably at regular intervals.

The present invention also relates to a method for monitoring the progression of fibrotic NASH in a subject, wherein said method comprises:

a) determining a score by measuring in a sample of said subject CK-18, AST and HOMA and combining the measures of CK-18, AST and HOMA in a mathematical function as described hereinabove;
b) comparing the score to a personalized reference value.

The term "reference value" is defined hereinabove. According to the present invention, the reference value is a personalized reference value. In one embodiment, the personalized reference value for a subject is the first score value obtained for said subject.

In one embodiment, said method comprises:

- determining a score by measuring in a sample of said subject CK-18, AST and HOMA and combining the measures of CK-18, AST and HOMA in a mathematical function as described hereinabove, comparing the score value to a reference value as described hereinabove;
- repeating the determination of the score at least twice, and preferably at regular intervals.

In one embodiment, the method for monitoring the progression of fibrotic NASH, in a subject comprises:

- determining a score by measuring in a sample of said subject CK-18, AST and HOMA and combining the measures of CK-18, AST and HOMA in a mathematical function as described hereinabove;
- repeating the determination of the score at least twice, and preferably at regular intervals;
- comparing the scores obtained;

thereby monitoring the progression of fibrotic NASH in the subject.

In one embodiment, a score is determined at least twice (such as, for example, twice, three times, four times or more) over a period of time. In one embodiment, the score is determined once a week, once a month or once a year. In one embodiment, the determination of the score is repeated every 6 months, every 9 months or every 18 months. In another embodiment, the determination of the score is repeated every year, every 2 years, every 3 years, every 4 years or every 5 years.

Another object which is outside the subject-matter of the claims is at least one therapeutic agent for use in the treatment of NASH, preferably fibrotic NASH in a subject, wherein the subject to be treated is identified by:

- measuring in a sample of said subject CK-18, AST and HOMA; and
- combining the measures of CK-18, AST and HOMA in a mathematical function to obtain a score value as described hereinabove, optionally comparing the score value to at least one reference value as described hereinabove.

In one embodiment which is outside the subject-matter of the claims, the at least one therapeutic agent is selected from the group comprising: drugs regulating metabolism homeostasis (such as, for example, thiazolidine derivatives, obeticholic acid, elafibranor); drugs regulating oxidative stress (such as, for example, vitamin E, ask-1 inhibitor); drugs regulating inflammation (such as, for example, cenicriviroc); drugs regulating apoptosis (such as, for example, the caspase inhibitor Emricasan); and anti-fibrotic drugs (such as, for example, simtuzumab).

In one embodiment which is outside the subject-matter of the claims, the at least one therapeutic agent is selected from the group comprising insulin sensitizers (such as rosiglitazone, pioglitazone and MSDC-0602K); farnesoid X receptor (FXR) agonists (such as obeticholic acid (also referred to as OCA), GS-9674, LJN452, LMB763 and EDP-305); Peroxisome Proliferator-Activated Receptor $\alpha/\delta$ (PPAR $\alpha/\delta$) agonists (such as elafibranor, saroglitazar and IVA337); fibroblast growth factor 19 (FGF19) analogs (such as NGM282); fibroblast growth factor 21 (FGF21) analogs (such as PF-05231023); recombinant FGF21 (such as BMS-986036); stearoyl-coenzyme A desaturase 1 (SCD1) inhibitors (such as aramchol); apical sodium-dependent bile acid transporter (ASBT) inhibitors (such as volixibat); acetyl-coA carboxylase (ACC) inhibitors (such as GS-0976); glucagon-like peptide-1 (GLP-1) analogs (such as liraglutide, semaglutide exenatide and taspoglutide); ursodeoxycholic acid and norursodeoxycholic acid (NorUDCA); taurine; polyenephosphatidylcholine; thyroid hormone receptor (THR) $\beta$-agonists (such as MGL-3196); antioxidant agents (such as vitamin E and vitamin C); apoptosis signal-regulating kinase 1 (ASK1) inhibitors (such as GS-4997); DPP-4 inhibitors (such as sitagliptin, alogliptin, vildagliptin, saxagliptin, and linagliptin); vascular adhesion protein-1 (VAP-1) inhibitors (such as PXS-4728A); phosphodiesterase-4 (PDE-4) inhibitors; angiotensin II-1 type receptor antagonists (such as losartan); anti-inflammatory compounds (such as cenicriviroc, VLX-103 (oral pentamidine) and hyperimmune bovine clostrum); Toll-like receptor 4 antagonists (such as nalmefene); caspase inhibitors (such as emricasan); anti-fibrotic compounds (such as simtuzumab and GR-MD-02); pentoxifylline; S-adenosylmethionine; milk thistle; and probiotics.

In one embodiment which is outside the subject-matter of the claims, the at least one therapeutic agent is selected from the group comprising obeticholic acid; elafibranor; cenicriviroc; ask-1 inhibitor; thiazolidine derivatives; and vitamin E. In one embodiment which is outside the subject-matter of the claims, the at least one therapeutic agent is selected from the group comprising thiazolidine derivatives, preferably pioglitazone or rosiglitazone; antioxidants, preferably vitamin E and/or vitamin C; obeticholic acid; elafibranor; cenicriviroc; and ask-1 inhibitor; and any combination thereof. In another embodiment, the at least one therapeutic agent is obeticholic acid or elafibranor.

The present disclosure also relates to a method for monitoring treatment response in a subject treated for NASH, preferably fibrotic NASH, wherein said method comprises measuring cytokeratin 18 (CK-18), aspartate aminotransferase (AST) and Homeostasis model assessment of insulin resistance (HOMA) in a sample of the subject at least twice, and preferably at regular intervals, said method not being encompassed by the subject-matter of the claims.

According to the present invention, in a method for monitoring treatment response in a subject treated for fibrotic NASH, the measure is performed at least once before the beginning of the treatment and at least once after the beginning of the treatment.

In another embodiment which is not encompassed by the subject-matter of the claims, the method comprises:

a) determining a score before the beginning of the treatment by measuring in a sample of said subject CK-18, AST and HOMA and combining the measures of CK-18, AST and HOMA in a mathematical function as described hereinabove;

b) repeating the determination of the score after the beginning of the treatment.

In one embodiment which is not encompassed by the subject-matter of the claims, the method comprises comparing the measures or the scores obtained to a reference value.

The term "reference value" is defined hereinabove. In one embodiment which is outside the subject-matter of the claims, the reference value is a personalized reference value. According to the present invention, the personalized reference value for a subject is the score value obtained for said subject before the beginning of treatment.

Thus, in one embodiment which is outside the subject-matter of the claims, the method comprises:

- measuring CK-18, AST and HOMA in a sample of the subject before the beginning of the treatment; and
- repeating the measures after the beginning of the treatment; and
- comparing the measures obtained before and after the beginning of the treatment.

The method for monitoring treatment response in a subject treated for fibrotic NASH comprises:

a) determining a score before the beginning of the treatment by measuring in a sample of said subject CK-18, AST and HOMA and combining the measures of CK-18, AST and HOMA in a mathematical function as described hereinabove;

b) repeating the determination of the score after the beginning of the treatment; and

c) comparing the scores obtained before and after the beginning of the treatment.

According to the present invention, the method for monitoring treatment response in a subject treated for fibrotic NASH, comprises:

a) determining a score before the beginning of the treatment by measuring in a sample of said subject CK-18, AST and HOMA and combining the measures of CK-18, AST and HOMA in a mathematical function as described hereinabove;

b) repeating the determination of the score after the beginning of the treatment; and

c) comparing the scores obtained before and after the beginning of the treatment;

thereby determining whether said treatment is effective.

In one embodiment, the determination of the score after the beginning of the treatment is repeated at regular intervals. Thus, in one embodiment, the determination of the score is repeated every month, every 2 months, every 3 months, every 6 months or every 9 months after the beginning of the treatment. In another embodiment, the determination of the score is repeated every year, every 2 years, every 3 years, every 4 years or every 5 years after the beginning of the treatment.

In one embodiment, a significant decrease in a score determined after the beginning of the treatment as mentioned hereinabove as compared to the score determined before the beginning of the treatment indicates that the treatment is effective. In one embodiment, a significant decrease in a score is a decrease of at least about 2,5%, 5%, 7.5%, 10%, 12.5%, 15%, 17.5%, 20%, 22.5%, 25%, 27.5%, 30%, 32.5%, 35%, 37.5%, 40%, 42.5%, 45%, 47.5% or 50% as compared to the score determined before the beginning of the treatment. In another embodiment, a significant decrease in a score is a decrease greater than about 2,5%, 5%, 7.5%, 10%, 12.5%, 15%, 17.5%, 20%, 22.5%, 25%, 27.5%, 30%, 32.5%, 35%, 37.5%, 40%, 42.5%, 45%, 47.5% or 50% as compared to the score determined before the beginning of the treatment.

The method of the invention specifically allows to assess the presence of fibrotic NASH. Indeed, the Applicant demonstrated that the new score (that may also be referred to as MACK-3 test), combining AST, HOMA and CK-18 in a binary logistic regression, has a very good accuracy for the non-invasive diagnosis of fibrotic NASH with 93.3% of correctly classified patients, a sensitivity of 90.0%, and a specificity of 94.2%, and with AUROC at $0.846 \pm 0.016$. AUROC (area under the ROC curve) is an indicator of the accuracy of a diagnostic test commonly calculated to assess the performance of diagnostic tests. Usually, it is considered that AUROC between 0.5 and 0.7 indicates that the test has poor to fair discrimination. By contrast, AUROC between 0.7 and 0.8 indicates that the test has acceptable discrimination and AUROC between 0.8 and 0.9 indicates that the test has excellent discrimination (Forthofer RN, Lee ES, Hernandez M (2006) Biostatistics: A guide to Design, Analysis, and Discovery: Elsevier).

Furthermore, the results presented in the Examples demonstrate that the MACK-3 test has a significantly superior diagnostic accuracy compared to that of previously described tests. In particular, MACK-3 is more accurate than the NALFD Fibrosis Score (NFS) and the NASH-score of WO2014/049131, both for NASH and for fibrotic NASH. Indeed, for the diagnostic of NASH, MACK-3 has an AUROC of $0.723 \pm 0.018$ (see Table 13) whereas NFS has an AUROC of $0.521 \pm 0.020$ (see Table 4) and NASH-score has an AUROC of $0.498 \pm 0.020$ (see Table 13). For the diagnostic of fibrotic NASH, MACK-3 has an AUROC of $0.846 \pm 0.016$ (see Tables 8 and 14) whereas NFS has an AUROC of $0.654 \pm 0.023$ (see Tables 4 and 8) and NASH-score has an AUROC of $0.647 \pm 0.023$ (see Table 14).

The MACK-3 test also displays a significantly superior diagnostic accuracy compared to that of untested combinations disclosed in WO2014/049131, *i.e.,* the combinations of BMI, CK-18 and glycemia (combination B); and CK-18, weight and insulin (combination C). The diagnostic accuracy of the MACK-3 test is also significantly superior to that of the combinations of CK-18, BMI, and HOMA (combination A). Indeed, for the diagnostic of fibrotic NASH, MACK-3 has an AUROC of $0.846 \pm 0.016$ (see Tables 8, 14 and 15) whereas combination A has an AUROC of $0.757 \pm 0.021$, combination B has an AUROC of $0.784 \pm 0.019$ and combination C has an AUROC of $0.736 \pm 0.022$ (see Table 15). As indicated in Table 15, the differences in diagnostic accuracy between MACK-3 and combinations A, B and C are significant with a p-value lower than 0.050.

Thus, for the diagnostic of fibrotic NASH, the MACK-3 test displays a surprising and significant superiority in diagnostic accuracy when compared to several combinations of markers described in WO2014/049131.

Finally, the MACK-3 test displays a significantly superior diagnostic accuracy compared to that of the CHA index as described in Polyzos SA et al. Ann Hepatol. 2013 Sep-Oct;12(5):749-57. Indeed, for the diagnostic of fibrotic NASH, the overall diagnostic accuracy of MACK-3 is about 93% of patients correctly classified whereas that of the CHA index is about 75.5% of patients correctly classified (see Table 17). In particular, the MACK-3 test is characterized by both a significantly higher sensitivity (78.3% vs 90%, respectively, in the validation set, p = 0.016) and a significantly higher specificity (74.4% vs 94.2%, respectively, in the validation set, p < 0.001) when compared to the CHA index.

Thus, for the diagnostic of fibrotic NASH, the MACK-3 test also displays a surprising and significant superiority in diagnostic accuracy when compared to the CHA index as described in Polyzos SA et al. Ann Hepatol. 2013 Sep-Oct;12(5):749-57.

Fibrotic NASH, as defined in the present invention, is a particularly relevant diagnostic target in the clinical practice. Indeed, among the patients suffering from NAFLD or NASH, patients with fibrotic NASH particularly stand to benefit from therapeutic treatment since their liver injuries, while significant, can still be reversed. The significance of fibrotic NASH is illustrated by the fact that criteria defining fibrotic NASH, *i.e.,* an active NASH with a NAS $\geq 4$ and a significant fibrosis (fibrosis stage F $\geq 2$, preferably according to the NASH-CRN staging system), are the inclusion criteria of on-going clinical trials aiming to assess the safety and efficacy of therapeutic agents in subjects with NASH. Moreover, fibrotic NASH is now used in the stratification of NAFLD severity by the latest international EASL-EASD-EASO Clinical Practice Guidelines (EASL. EASL-EASD-EASO Clinical Practice Guidelines for the management of non-alcoholic fatty liver disease. J Hepatol 2016;64:1388-1402).

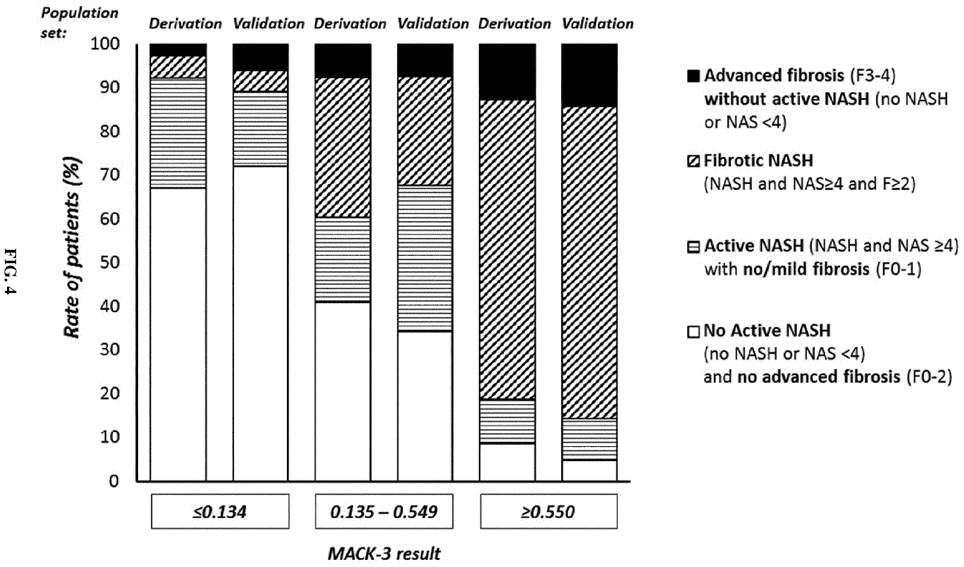

The method of the invention thus represents a simple and accurate solution to improve the screening and monitoring of patients for therapeutic trials in NASH. Indeed, inclusions in phase III therapeutic trials in the setting of NASH are currently challenged by high rates of screening failure. Since it allows the identification of a subset of NAFLD patients with a high rate of fibrotic NASH (see Figure 4), the MACK-3 test should considerably reduce the rate of screening failure. Furthermore, the method of the invention allows the non-invasive identification and monitoring of the patients who are likely to benefit the most from the newly developed NASH therapies.

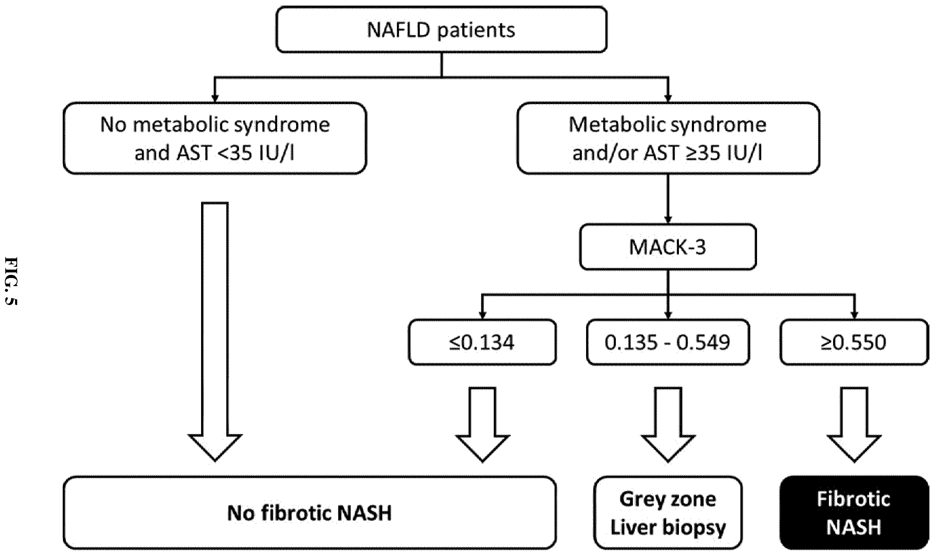

NAFLD now concerns 25% of the general population (Younossi ZM et al. Hepatology 2016;64:73-84). Consequently, with the upcoming arrival of new treatments, many patients will need to be tested for the presence of fibrotic NASH. One of the methods of the invention (also referred to as MACK-3 algorithm) allows to limit extensive measurements of specialized biomarkers in patients with NAFLD (see Figure 5) and thus to limit costs related to the use of the MACK-3 test in clinical practice.

**BRIEF DESCRIPTION OF THE DRAWINGS**

**Figure 1** is a diagram showing the prevalence of liver lesions in the study population of 846 patients of Example 1 (NASH: nonalcoholic steatohepatitis; NAS: NAFLD Activity Score).

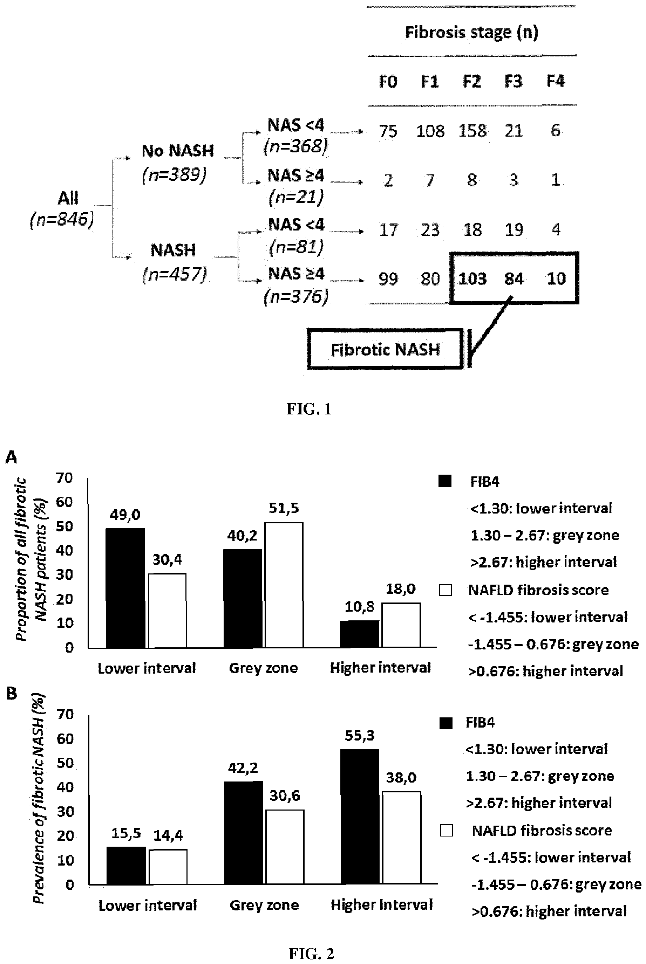

**Figure 2** is a combination of graphs showing the accuracy for the diagnosis of fibrotic NASH of the NAFLD fibrosis score (NFS) and the FIB4 test, each performed with their published diagnostic cut-offs: for NFS -1.455 and 0.676, as described in Angulo P et al., Hepatology 2007;45:846-54 and for FIB4 1.30 and 2.67, as described in Shah AG et al., Clin Gastroenterol Hepatol 2009;7:1104-12. The accuracy of the NFS and FIB4 tests is assessed through the determination **(A)** of the proportion of all fibrotic NASH patients included in the different intervals of the NFS and FIB4 tests; and **(B)** of the prevalence of fibrotic NASH as a function of the diagnostic interval of the NFS and FIB4 tests.

**Figure 3** is a combination of graphs showing the CK-18 level of patients (expressed in IU/L) as a function of the presence in the patients of: NASH and/or NAS $\geq$ 4 **(A)** or NASH and/or NAS $\geq$ 4 with fibrosis stage F $\geq$ 2, the latter defined as either fibrosis stage F0-1 or fibrosis stage F2-4 **(B).**

**Figure 4** is a graph showing the prevalence of the NAFLD lesions (advanced fibrosis without active NASH; fibrotic NASH; active NASH with no/mild fibrosis; and no active NASH and no advanced fibrosis) as a function of the three intervals of MACK-3 score in both the derivation and the validation population sets.

**Figure 5** is a diagram illustrating the MACK-3 algorithm for the diagnosis of fibrotic NASH in clinical practice.

**Figure 6** is a graph showing the prevalence of the NAFLD lesions (advanced fibrosis without active NASH; fibrotic NASH; active NASH with no/mild fibrosis; and no active NASH and no advanced fibrosis) as a function of the four subgroups defined by the MACK-3 algorithm.

**EXAMPLES**

The present invention is further illustrated by the following examples.

**Example 1:**

***Patients and methods***

Patients

The study population was obtained by pooling the data from three published studies performed in Angers (Boursier J et al., J Hepatol. 2016 Sep;65(3):570-8), Antwerp (Francque SM et al., Clin Gastroenterol Hepatol 2012;10:1162-8) and Nice (Anty R et al., Aliment Pharmacol Ther 2010;32:1315-22). To be included, patients had to have biopsy-proven NAFLD as defined by the presence of liver steatosis after exclusion of concomitant steatosis-inducing drugs (such as corticosteroids, tamoxifen, amiodarone, or methotrexate), excessive alcohol consumption (> 30 g/day in men or > 20 g/day in women), chronic hepatitis B or C infection, and histological evidence of other concomitant chronic liver disease. Patients were excluded if they had cirrhosis complication (hepatic encephalopathy, ascites, variceal bleeding, systemic infection, or hepatocellular carcinoma). The recruitment was different among the three centers: Angers center included outpatients from the hepatology department, Antwerp center included obese patients without pre-existing diabetes who presented at the obesity clinic for a problem of overweight, and Nice center included morbidly obese patients referred for a bariatric surgery. Metabolic syndrome was defined as the presence of at least three of the following parameters (Alberti KG et al., Circulation 2009;120:1640-1645): elevated waist circumference ($\geq$94 cm in men or $\geq$80 cm in women), elevated blood pressure (systolic blood pressure $\geq$130 mm Hg or diastolic blood pressure $\geq$85 mm Hg or antihypertensive drug), elevated glycemia ($\geq$100 mg/dL or anti-diabetic drug), elevated triglycerides ($\geq$150 mg/dL or lipid-lowering drug), low HDL cholesterol (<40 mg/dL in men or <50 mg/dL in women or lipid-lowering drug).

Histology

Liver biopsy was performed percutaneously (16-gauge Menghini) in Angers, percutaneously (16-gauge Menghini) or peri-operatively (14-gauge Tru-Cut) in Antwerp, or during bariatric surgery (hepatic wedges) in Nice. Pathological examination of liver biopsies was performed by one (Angers, Antwerp) or two (Nice) senior expert(s) specialized in hepatology who were blinded for patient data. Steatosis, lobular inflammation and ballooning were semi-quantitatively graded using the NASH-CRN scoring system (Kleiner DE et al., Hepatology 2005;41:1313-21). The NAS, ranging from 0 to 8, corresponded to the sum of the scores for steatosis, lobular inflammation and ballooning (Brunt EM et al., Hepatology 2011;53:810-20). In accordance with the latest recommendations (Sanyal AJ et al., Hepatology 2011;54:344-53; Sanyal AJ et al., Hepatology 2015;61:1392-405), NASH was defined as the presence of a score $\geq$ 1 for each of the three components of the NAS. "Active NASH" was defined as the presence of NASH with a NAS score $\geq$ 4. Liver fibrosis was staged from F0 to F4 according to the NASH-CRN scoring system (Kleiner DE et al., Hepatology 2005;41:1313-21).

Significant fibrosis was defined as F $\geq$ 2 and advanced fibrosis as F $\geq$ 3. Finally, "fibrotic NASH" was defined as the presence of an active NASH with significant fibrosis, *i.e.,* NASH and NAS $\geq$ 4 and fibrosis F $\geq$ 2. Fibrotic NASH was the primary diagnostic target of the study.

A subgroup of 30 patients covering the histological spectrum of NAFLD lesions was selected in the Angers population to study the inter-center agreement on histopathological evaluation of liver lesions. Liver biopsies were digitized using an Aperio digital slide scanner (Scanscope CS System, Aperio Technologies, Vista CA 92081, USA) image processor that provided high quality images (30,000 x 30,000 pixels) at a resolution of 0.5 $\mu$m/pixel (magnification x20). Two slides were digitized for each patient: one stained with hematoxylin and eosin and one with picrosirius red. The digitized slides were sent to the expert pathologist from each of the three investigating centers to score steatosis, lobular inflammation, ballooning, and fibrosis according to the NASH-CRN scoring system.

Blood parameters

All blood samples from the three original studies were taken in fasting conditions (Boursier J et al., J Hepatol. 2016 Sep;65(3):570-8; Francque SM et al., Clin Gastroenterol Hepatol 2012;10:1162-8; Anty R et al., Aliment Pharmacol Ther 2010;32:1315-22).

The data available from the three studies enabled the calculation of the following blood fibrosis tests according to published formulas: BARD (Harrison SA et al., Gut 2008;57:1441-7), FIB4 (Sterling RK et al., Hepatology 2006;43:1317-25), and NAFLD Fibrosis Score (NFS) (Angulo P et al., Hepatology 2007;45:846-54). Serum levels of insulin were measured on fresh samples using Chimiluminescence 12000 (Abbott) in Angers, Cobas Instrument (Roche) in Antwerp, and Centaur XP (Siemens) in Nice. HOMA was calculated as follows: fasting glucose (mmol/L) * fasting insulin ($\mu$U/mL) / 22.5. To limit the influence of supra-physiological results of insulinemia induced by insulin therapy, HOMA was capped at 10. Serum level of apoptotic caspase-3 generated CK-18 fragments was measured on frozen samples stored at -80°C in each center, using the M30-Apoptosense enzyme-linked immunosorbent assay kit (PEVIVA, Bromma, Sweden). All blood assays were performed in the laboratories of the three participating centers. Blood fibrosis tests demonstrably have excellent inter-laboratory reproducibility (Cales P et al., Clin Biochem 2008;41:10-18).

Statistics

Quantitative variables were expressed as mean $\pm$ standard deviation. Inter-observer agreement of the histological scoring was determined using the Fleiss' Kappa score (Shrout PE, Fleiss JL. Psychol Bull 1979;86:420-428). Correlations between quantitative variables were determined by using the Spearman correlation coefficient (Rs). Accuracy of non-invasive tests was evaluated using the Area Under the Receiver Operating Characteristics (AUROC) and compared between tests using the Delong test. Statistical analyses were performed using SPSS version 18.0 software (IBM, Armonk, NY, USA) and SAS 9.1 (SAS Institute Inc., Cary, NC, USA).

**Development of the new blood test for the diagnosis of fibrotic NASH** - To facilitate future automatic calculation by laboratory systems, only biological parameters were considered for the development of the new blood test dedicated to the diagnosis of fibrotic NASH. Variables with skewed distribution were log-transformed (natural logarithm). The study population was randomly divided into derivation and validation sets. In the derivation set, the statistically significant variables identified by the univariate analysis ($p < 0.05$) were introduced in a multivariate analysis whose regression formula (binary logistic regression) was used to develop the new blood test. The diagnostic accuracy of the new test was then evaluated in the validation set.

**Sample size calculation** - With $\alpha$ risk: 5%, $\beta$ risk: 10%, fibrotic NASH prevalence: 25%, expected AUROC for fibrotic NASH: 0.70 for the best blood fibrosis test and 0.85 for the new test, AUROC correlation: 0.30 and bilateral testing, the required sample size for the validation set was 264 patients. The whole study population (n=846) was therefore 2:1 randomly divided so that the validation set included 282 patients.

***Results***

Patient characteristics

846 patients were included in the study. Their characteristics are detailed in **Table 1** below. 62.1% were female and mean age was 47.5 $\pm$ 13.6 years.

**Table 1:** Patient characteristics at inclusion

| | All (n=846) | Angers (n=309) | Antwerp (n=270) | Nice (n=267) | p[a] |
|---|---|---|---|---|---|
| Age (years) | 47.5 ± 13.6 | 56.1 ± 12.4 | 44.9 ± 12.3 | 40.3 ± 10.7 | < 0.001 |
| Male sex (%) | 37.9 | 60.8 | 37.8 | 11.6 | < 0.001 |
| BMI (kg/m$^2$) | 38.5 ± 7.6 | 32.5 ± 6.0 | 40.0 ± 6.6 | 43.8 ± 4.8 | < 0.001 |
| Elevated waist circumference (%) [b] | 98.5 | 95.8 | 100.0 | 100.0 | < 0.001 |
| Elevated blood pressure (%) [c] | 68.2 | 79.5 | 69.7 | 51.3 | < 0.001 |
| Elevated glycemia (%) [d] | 47.8 | 75.7 | 19.3 | 44.4 | < 0.001 |
| Elevated triglycerides (%) [e] | 49.4 | 60.4 | 48.7 | 37.5 | < 0.001 |
| Reduced HDL cholesterol (%) [f] | 54.9 | 68.4 | 57.8 | 36.1 | < 0.001 |
| Metabolic syndrome (%) [g] | 67.7 | 83.1 | 62.8 | 54.4 | < 0.001 |
| Diabetes (%) [h] | 27.2 | 53.4 | 3.3 | 21.1 | < 0.001 |
| Biopsy length (mm) | 24 ± 12 | 30 ± 12 | 16 ± 8 | - i | < 0.001 |
| Steatosis (0/1/2/3, %) | 4.1/42.4/29.0/24.5 | 5.8/46.3/29.1/18.8 | 0.0/44.1/31.5/24.4 | 6.4/36.3/26.2/31.1 | < 0.001 |
| Lobular inflammation (0/1/2/3, %) | 38.7/44.8/13.0/3.5 | 17.2/69.9/12.9/0.0 | 18.9/44.4/25.6/11.1 | 83.5/16.1/0.4/0.0 | < 0.001 |
| Ballooning (0/1/2) | 40.1/37.7/22.2 | 23.0/50.2/26.9 | 17.8/44.1/38.1 | 82.4/16.9/0.7 | < 0.001 |
| NAS | 3.4 ± 1.8 | 3.6 ± 1.5 | 4.3 ± 1.8 | 2.2 ± 1.4 | < 0.001 |
| NASH (%) | 54.0 | 70.2 | 72.6 | 16.5 | < 0.001 |
| Fibrosis stage (0/1/2/3/4, %) | 22.8/25.8/33.9/15.0/2.5 | 8.4/26.2/29.8/29.4/6.1 | 60.4/18.1/12.6/8.1/0.7 | 1.5/33.0/60.3/5.2/0.0 | < 0.001 |
| Fibrotic NASH (%)[j] | 23.3 | 42.1 | 15.9 | 9.0 | < 0.001 |
| AST (IU/L) | 36 ± 21 | 45 ± 24 | 33 ± 18 | 27 ± 16 | < 0.001 |
| ALT (IU/L) | 50 ± 34 | 64 ± 38 | 48 ± 28 | 35 ± 28 | < 0.001 |
| GGT (IU/L) | 71 ± 97 | 118 ± 141 | 44 ± 33 | 43 ± 40 | < 0.001 |
| Bilirubin (μmol/L) | 10 ± 5 | 11 ± 6 | 10 ± 4 | 8 ± 4 | < 0.001 |
| Prothrombin time (%) | 95 ± 11 | 98 ± 12 | 94 ± 10 | 92 ± 9 | < 0.001 |
| Platelets (G/L) | 263 ± 73 | 224 ± 67 | 294 ± 66 | 278 ± 66 | < 0.001 |
| CK-18 (IU/L) | 272 ± 257 | 289 ± 321 | 264 ± 243 | 260 ± 172 | 0.082 |

(continued)

|  | All (n=846) | Angers (n=309) | Antwerp (n=270) | Nice (n=267) | p[a] |
|---|---|---|---|---|---|
| HbAlc (%) | 6.1 ± 1.2 | 6.6 ± 1.3 | 5.7 ± 0.6 | 6.0 ± 1.2 | < 0.001 |

BMI: body mass index; AST: aspartate aminotransferase; ALT: alanine aminotransferase; GGT: gamma glutamyl-transpeptidase; HbA1c: hemoglobin A1c also known as glycated hemoglobin.

[a] by Kruskal Wallis test; [b] waist circumference $\geq$ 94 cm in men or $\geq$ 80 cm in women; [c] systolic blood pressure $\geq$ 130 mm Hg or diastolic blood pressure $\geq$ 85 mm Hg or antihypertensive drug; [d] glycemia $\geq$ 100 mg/dL or anti-diabetic drug; [e] triglycerides $\geq$ 150 mg/dL or lipid-lowering drug; [f] HDL cholesterol < 40 mg/dL in men or < 50 mg/dL in women or lipid-lowering drug; [g] defined as $\geq$ 3 parameters present among following: elevated waist circumference, elevated blood pressure, elevated glycemia, elevated triglycerides, low HDL cholesterol; [h] glycemia $\geq$ 126 mg/dL or anti-diabetic drug; [i] surgical samples; [j] defined as the presence of NASH and NAS $\geq$ 4 and fibrosis F $\geq$ 2.

Two third of patients (67.7%) had a metabolic syndrome (MetS) and 27.2% were diabetics. In Angers and Antwerp, mean liver biopsy length was 24 ± 12mm and 91.0% of biopsies were ≥ 10mm, 73.9% were ≥15mm and 60.8% were ≥20mm. NASH was present in 54.0% of patients, NAS ≥ 4 in 46.9% and fibrosis F ≥ 2 in 51.4%. Finally, 23.3% had fibrotic NASH as defined by the presence of all the three criteria NASH, NAS ≥ 4 and F ≥ 2 (see **Figure 1).** Because the recruitment sources were not the same, patient characteristics significantly differed between the three centers.

Inter-observer agreement on histopathological evaluation of liver lesions

The liver biopsies of 30 patients were all read by the expert pathologist from each investigating center, for a total of 90 observations. The distribution of the recorded scores is shown in **Table 2** below. Fleiss' Kappa score was 0.77 for steatosis, 0.62 for lobular inflammation, 0.64 for ballooning, 0.58 for NASH, 0.81 for the fibrosis stage, and 0.60 for fibrotic NASH. As shown in **Table 3** below, these results were very close to what has been previously observed between nine experts of the NASH-CRN.

**Table 2:** Distribution of liver lesions in the 90 observations of the inter-observer reproducibility study

| Liver lesion | Distribution |
|---|---|
| Steatosis (0/1/2/3, %) | 11.1 / 37.8 / 24.4 / 26.7 |
| Lobular inflammation (0/1/2/3, %) | 34.4 / 48.9 / 15.6 / 1.1 |
| Ballooning (0/1/2, %) | 26.7 / 47.8 / 25.6 |
| NASH (%) | 51.1 |
| NAS (mean ± SD) | 3.5 ± 2.0 |
| Fibrosis stage (0/1/2/3/4, %) | 11.1 / 25.6 / 26.7 / 23.3 / 13.3 |
| Fibrotic NASH (%) | 45.6 |

**Table 3:** Inter-observer agreement on histopathological examination of liver lesions

| Liver lesion | Kappa score | |
|---|---|---|
| | Study result | Kleiner study [a] |
| Steatosis | 0.77 | 0.79 |
| Lobular inflammation | 0.62 | 0.45 |
| Ballooning | 0.64 | 0.56 |
| NASH | 0.58 | 0.61 |
| Fibrosis stage | 0.81 | 0.84 |
| Fibrotic NASH | 0.60 | - |
| [a] The Kleiner study (Kleiner DE, et al. Hepatology 2005;41:1313-1321) evaluated the inter-observer agreement between nine expert pathologists from the NASH CRN | | |

Accuracy of blood fibrosis tests for the evaluation of NAFLD severity and for the diagnosis of fibrotic NASH

As shown in **Table 4** below, blood fibrosis tests performed better in diagnosing high fibrosis stages, *i.e.,* advanced fibrosis and cirrhosis. On the contrary, they were poorly accurate for the diagnosis of NASH or NAS ≥ 4 with AUROC no higher than 0.65. For the diagnosis of fibrotic NASH, FIB4 had a higher AUROC (0.732 ± 0.021) than NFS (0.654 ± 0.023, p < 0.001) and BARD (0.566 ± 0.023, p < 0.001). Two diagnostic cut-offs are published for NFS (-1.455 and 0.676) and for FIB4 (1.30 and 2.67) in NAFLD (Angulo P et al., Hepatology 2007;45:846-54; Shah AG et al., Clin Gastroenterol Hepatol 2009;7:1104-12). As shown in **Figure 2,** the low cut-off provided only poor to moderate sensitivity for fibrotic NASH: 49.0% of the patients with fibrotic NASH were included in the lower interval of the FIB4 (< 1.30) and 30.4% in the lower interval of the NFS (< -1.455). Additionally, their high cut-offs identified very few patients with fibrotic NASH (NFS: 18.0%, FIB4: 10.8%) and had poor positive predictive value (NFS: 38.0%, FIB4: 55.3%) as shown in **Figure 2.** Taken together, all these results suggest that BARD, FIB4, and NFS are not optimal blood tests for the detection of fibrotic NASH in NAFLD patients.

**Table 4:** AUROC of blood fibrosis tests and CK-18 for the diagnosis of NAFLD lesions

| Diagnostic target | BARD | NFS | FIB4 | CK-18 |
|---|---|---|---|---|
| Mild fibrosis (F $\geq$ 1) | 0.626 $\pm$ 0.022 | 0.721 $\pm$ 0.020 | 0.653 $\pm$ 0.021 | 0.607 $\pm$ 0.022 |
| Significant fibrosis (F $\geq$ 2) | 0.641 $\pm$ 0.019 | 0.686 $\pm$ 0.018 | 0.648 $\pm$ 0.019 | 0.634 $\pm$ 0.019 |
| Advanced fibrosis (F $\geq$ 3) | 0.633 $\pm$ 0.025 | 0.720 $\pm$ 0.023 | 0.808 $\pm$ 0.021 | 0.659 $\pm$ 0.027 |
| Cirrhosis (F4) | 0.648 $\pm$ 0.065 | 0.865 $\pm$ 0.036 | 0.874 $\pm$ 0.040 | 0.572 $\pm$ 0.061 |
| NASH | 0.466 $\pm$ 0.020 | 0.521 $\pm$ 0.020 | 0.651 $\pm$ 0.019 | 0.595 $\pm$ 0.019 |
| NAS $\geq$ 4 | 0.433 $\pm$ 0.020 | 0.490 $\pm$ 0.020 | 0.604 $\pm$ 0.019 | 0.643 $\pm$ 0.019 |
| NASH and NAS $\geq$ 4 | 0.449 $\pm$ 0.020 | 0.493 $\pm$ 0.020 | 0.597 $\pm$ 0.020 | 0.638 $\pm$ 0.019 |
| Fibrotic NASH[a] | 0.566 $\pm$ 0.023 | 0.654 $\pm$ 0.023 | 0.732 $\pm$ 0.021 | 0.715 $\pm$ 0.022 |
| NAS: NAFLD Activity Score [a] defined as the presence of NASH and NAS $\geq$ 4 and F $\geq$ 2 | | | | |

Accuracy of CK-18 for the evaluation of NAFLD severity and for the diagnosis of fibrotic NASH

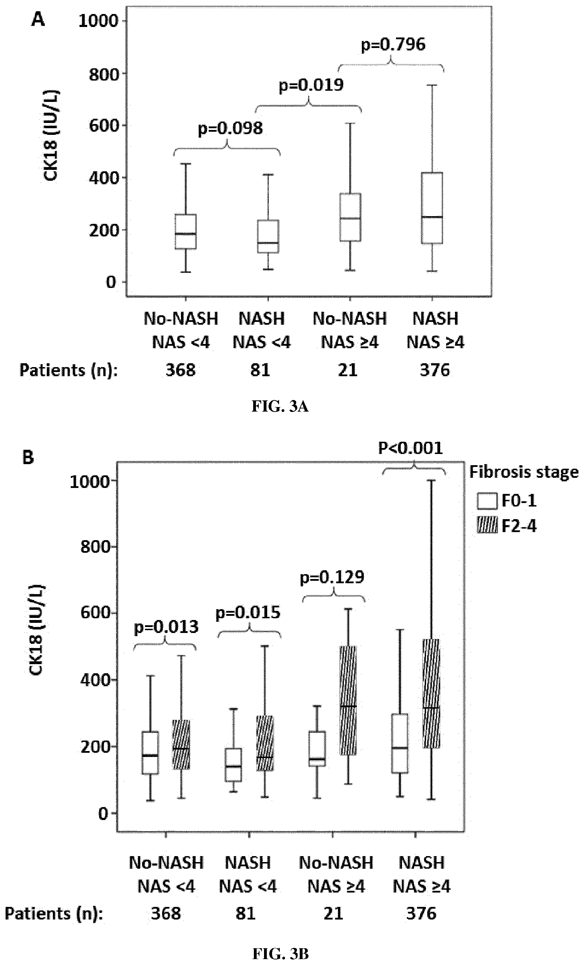

As shown in **Table 5** below, CK-18 was significantly correlated to steatosis, lobular inflammation, ballooning and fibrosis with significant difference between most of the adjacent grades/stages. In addition, CK-18 was significantly higher in patients having NASH compared to those without (respectively: 319 $\pm$ 316 vs 217 $\pm$ 142 IU/L, p < 0.001). Despite this, as illustrated in **Table 4** hereinabove, CK-18 surprisingly showed a low accuracy for the diagnosis of NASH with an AUROC at 0.595 $\pm$ 0.019. The level of CK-18 as a function of NASH, NAS $\geq$ 4 and F $\geq$ 2 was then evaluated. As illustrated in **Figure 3A,** in both NAS < 4 and NAS $\geq$ 4 subgroups, CK-18 level did not significantly differ between patients with NASH and patients without NASH. Interestingly, in the four subgroups defined by NASH and NAS $\geq$ 4, the level of CK-18 was significantly higher in F $\geq$ 2 patients compared to F0-1 patients **(Figure 3B).** Consequently, as seen in **Table 4** hereinabove, rather than a biomarker for the single diagnosis of NASH, CK-18 was more globally associated with the severity of NAFLD. CK-18 performed better in diagnosing fibrotic NASH and appeared as a candidate biomarker for this diagnostic target.

**Table 5:** CK-18 level as a function of NAFLD lesions

| Liver lesion | Rs (p) | Grade/stage | | | | | P value | | | |
|---|---|---|---|---|---|---|---|---|---|---|
| | | 0 | 1 | 2 | 3 | 4 | 0 vs 1 | 1 vs 2 | 2 vs 3 | 3 vs 4 |
| Steatosis | 0.302 (< 0.001) | 188 $\pm$ 139 | 213 $\pm$ 156 | 274 $\pm$ 221 | 386 $\pm$ 382 | - | 0.182 | < 0.001 | < 0.001 | - |
| Lobular Inflammation | 0.159 (< 0.001) | 220 $\pm$ 144 | 269 $\pm$ 218 | 397 $\pm$ 454 | 411 $\pm$ 427 | - | 0.056 | 0.001 | 0.972 | - |
| Ballooning | 0.122 (< 0.001) | 220 $\pm$ 143 | 292 $\pm$ 257 | 332 $\pm$ 374 | - | - | 0.005 | 0.420 | - | - |
| NAS $\geq$ 4 | - | 210 $\pm$ 138 | 342 $\pm$ 332 | - | - | - | < 0.001 | - | - | - |
| NASH | - | 217 $\pm$ 142 | 319 $\pm$ 316 | - | - | - | < 0.001 | - | - | - |
| Fibrosis | 0.253 (< 0.001) | 210 $\pm$ 156 | 224 $\pm$ 164 | 273 $\pm$ 206 | 447 $\pm$ 466 | 269 $\pm$ 135 | 0.347 | < 0.001 | < 0.001 | 0.159 |
| Fibrotic NASH[a] | - | 223 $\pm$ 155 | 433 $\pm$ 413 | - | - | - | < 0.001 | - | - | - |
| Rs: Spearman correlation coefficient; NAS: NAFLD Activity Score [a] defined as the presence of NASH and NAS $\geq$ 4 and F $\geq$ 2 | | | | | | | | | | |

**Table 6:** Characteristics of the derivation and validation sets

| | All (n=846) | Derivation (n=564) | Validation (n=282) | p[a] |
|---|---|---|---|---|
| Age (years) | 47.5 ± 13.6 | 47.4 ± 13.6 | 47.8 ± 13.6 | 0.716 |
| Male sex (%) | 37.9 | 39.4 | 35.1 | 0.260 |
| BMI (kg/m$^2$) | 38.5 ± 7.6 | 38.5 ± 7.5 | 38.4 ± 7.6 | 0.720 |
| Elevated waist circumference (%)[b] | 98.5 | 98.0 | 99.6 | 0.117 |
| Elevated blood pressure (%)[c] | 68.2 | 69.1 | 66.3 | 0.423 |
| Elevated glycemia (%)[d] | 47.8 | 49.0 | 45.4 | 0.343 |
| Elevated triglycerides (%)[e] | 49.4 | 49.6 | 49.1 | 0.942 |
| Reduced HDL cholesterol (%)[f] | 54.9 | 54.7 | 55.2 | 0.941 |
| Metabolic syndrome (%)[g] | 67.7 | 67.9 | 67.2 | 0.873 |
| Diabetes (%)[h] | 27.2 | 27.0 | 27.7 | 0.870 |
| Steatosis (0/1/2/3, %) | 4.1/42.4/29.0/24.5 | 3.9/41.8/29.4/24.8 | 4.6/43.6/28.0/23.8 | 0.900 |
| Lobular inflammation (0/1/2/3, %) | 38.7/44.8/13.0/3.5 | 39.2/43.4/14.0/3.4 | 37.6/47.5/11.0/3.9 | 0.515 |
| Ballooning (0/1/2) | 40.1/37.7/22.2 | 41.3/36.3/22.3 | 37.6/40.4/22.0 | 0.475 |
| NAS | 3.4 ± 1.8 | 3.4 ± 1.8 | 3.4 ± 1.9 | 0.799 |
| NASH (%) | 54.0 | 52.7 | 56.7 | 0.273 |
| Fibrosis stage (0/1/2/3/4, %) | 22.8/25.8/33.9/15.0/2.5 | 23.6/25.5/33.5/14.5/2.8 | 21.3/26.2/34.8/16.0/1.8 | 0.796 |
| Fibrotic NASH (%)[j] | 23.3 | 23.9 | 22.0 | 0.547 |
| AST (IU/L) | 36 ± 21 | 35 ± 21 | 36 ± 22 | 0.987 |
| ALT (IU/L) | 50 ± 34 | 50 ± 34 | 50 ± 36 | 0.955 |
| GGT (IU/L) | 71 ± 97 | 73 ± 106 | 67 ± 76 | 0.979 |
| Bilirubin (μmol/L) | 10 ± 5 | 10 ± 5 | 10 ± 6 | 0.720 |
| Prothrombin time (%) | 95 ± 11 | 95 ± 12 | 95 ± 9 | 0.632 |
| Platelets (G/L) | 263 ± 73 | 264 ± 72 | 262 ± 75 | 0.653 |
| CK-18 (IU/L) | 272 ± 257 | 265 ± 227 | 286 ± 307 | 0.603 |
| HbA1c (%) | 6.1 ± 1.2 | 6.1 ± 1.2 | 6.1 ± 1.1 | 0.988 |

BMI: body mass index; AST: aspartate aminotransferase; ALT: alanine aminotransferase; GGT: gamma glutamyl-transpeptidase; HbA1c: hemoglobin A1c also known as glycated hemoglobin.

[a] by Kruskal Wallis test; [b] waist circumference ≥ 94 cm in men or ≥ 80 cm in women; [c] systolic blood pressure ≥ 130 mm Hg and/or diastolic blood pressure ≥ 85 mm Hg and/or antihypertensive drug; [d] glycemia ≥ 100 mg/dL or anti-diabetic drug; [e] triglycerides ≥ 150 mg/dL or lipid-lowering drug; [f] HDL cholesterol < 40 mg/dL in men or < 50 mg/dL in women or lipid-lowering drug; [g] defined as ≥ 3 parameters present among following: elevated waist circumference, elevated blood pressure, elevated glycemia, elevated triglycerides, low HDL cholesterol; [h] glycemia ≥ 126 mg/dL or anti-diabetic drug; [j] NASH and NAS ≥ 4 and fibrosis F ≥ 2

New blood test for the non-invasive diagnosis of fibrotic NASH

The 846 patients were 2:1 randomly divided into a derivation set (564 patients) and a validation set (282 patients), with patient characteristics not being significantly different in the two sets, as shown in **Table 6** above. To facilitate a future automatic calculation of the new test by laboratory systems, only biological parameters were tested in the derivation set. In the derivation set, multivariate analysis identified three variables as independent predictors of fibrotic NASH: AST, HOMA and CK-18 (see **Table 7** below). The regression formula (binary logistic regression) of the multivariate analysis was used to derive a new blood test combining these three parameters: the MACK-3 (HOMA, AST, CK-18) resulting in a score value ranging from 0 to 1. The MACK-3 thresholds corresponding to 90% sensitivity and 95% specificity for fibrotic NASH were calculated in the derivation population at, respectively, 0.134 and 0.550.

**Table 7:** Independent predictors of fibrotic NASH in the derivation set

| Variable | Univariate | Multivariate | | |
|---|---|---|---|---|
| | p | Step | p | OR (95% CI) |
| AST (ln IU/L) | <0.001 | 1st | <0.001 | 6.35 (3.53 - 11.40) |
| ALT (ln IU/L) | <0.001 | | - | |
| AST/ALT ratio (ln) | 0.585 | | - | |
| GGT (ln IU/L) | <0.001 | | - | |
| PAL (ln IU/L) | 0.664 | | - | |
| Bilirubin (ln μmol/L) | 0.036 | | - | |
| Albumin (g/L) | 0.993 | | - | |
| Platelets (G/L) | <0.001 | | - | |
| Prothrombin time (%) | 0.647 | | - | |
| Uric acid (ln IU/L) | <0.001 | | - | |
| Ferritin (ln μg/L) | <0.001 | | - | |
| usCRP (ln mg/L) | 0.559 | | - | |
| Creatinine (ln μmol/L) | 0.558 | | - | |
| Hemoglobin (g/dL) | 0.003 | | - | |
| Leucocytes (ln G/L) | 0.273 | | - | |
| CK18 (ln IU/L) | <0.001 | 3rd | 0.003 | 1.86 (1.23 - 2.81) |
| HBalc (%) | <0.001 | | - | |
| Insulin (ln μU/mL) | <0.001 | | - | |
| HOMA[a] (ln) | <0.001 | 2nd | <0.001 | 3.42 (2.24 - 5.22) |
| [a] capped at 10 and then expressed as natural logarithm (ln) | | | | |

In the validation set, MACK-3 had a significantly higher AUROC for the diagnosis of fibrotic NASH (0.847 $\pm$ 0.030) than BARD, NFS and FIB4 (p $\leq$ 0.002, see **Table 8** below). Thus, in the validation set, NFS and FIB4 showed only moderate diagnostic accuracy for fibrotic NASH with AUROC respectively at 0.618 $\pm$ 0.040 and 0.721 $\pm$ 0.0037. Similarly, in the whole population study, the new MACK-3 test combining HOMA, AST, CK-18 showed a very good accuracy for the non-invasive diagnosis of fibrotic NASH with AUROC at 0.846 $\pm$ 0.016. By contrast, the NFS and FIB4 tests only showed moderate diagnostic accuracy with AUROC respectively at 0.654 $\pm$ 0.023 and 0.732 $\pm$ 0.021 **(Table 8).**

**Table 8:** AUROC of MACK-3 and blood fibrosis tests for the diagnosis of fibrotic NASH

| Test | All | Derivation | Validation | p |
|---|---|---|---|---|
| BARD | 0.566 $\pm$ 0.023 | 0.584 $\pm$ 0.028 | 0.528 $\pm$ 0.042 | 0.267 |
| NFS | 0.654 $\pm$ 0.023 | 0.671 $\pm$ 0.027 | 0.618 $\pm$ 0.040 | 0.272 |

(continued)

| Test | All | Derivation | Validation | p |
|---|---|---|---|---|
| FIB4 | $0.732 \pm 0.021$ | $0.738 \pm 0.025$ | $0.721 \pm 0.037$ | 0.703 |
| MACK-3 | $0.846 \pm 0.016$ | $0.845 \pm 0.019$ | $0.847 \pm 0.030$ | 0.955 |
| Comparison (p) | | | | |
| BARD vs NFS | < 0.001 | 0.003 | 0.015 | - |
| BARD vs FIB4 | < 0.001 | < 0.001 | < 0.001 | - |
| BARD vs MACK-3 | < 0.001 | < 0.001 | < 0.001 | - |
| NFS vs FIB4 | < 0.001 | 0.007 | < 0.001 | - |
| NFS vs MACK-3 | < 0.001 | < 0.001 | < 0.001 | - |
| FIB4 vs MACK-3 | < 0.001 | < 0.001 | 0.002 | - |

48.3% of the patients had MACK-3 $\leq$ 0.134 (90% sensitivity threshold), 36.0% were in the intermediate grey zone (0.135-0.549), and 15.7% had MACK-3 $\geq$ 0.550 (95% specificity threshold). The prevalence of fibrotic NASH in these three intervals was, respectively: 4.7%, 25.0% and 71.4% (p < 0.001, **Figure 4).** MACK-3 $\leq$ 0.134 provided 90.0% sensitivity for fibrotic NASH, and MACK-3 $\geq$ 0.550 had 94.2% specificity. Among the patients included in the highest interval (MACK-3 $\geq$ 0.550), 85.7% had fibrotic NASH or advanced F3-4 fibrosis **(Figure 4).** Further testing, for example a liver biopsy, is required for the patients included in the grey zone between the 0.134 and 0.550 thresholds. MACK-3 showed an excellent diagnostic accuracy for fibrotic NASH with 93.3% well-classified patients as shown in **Table 9** below. All the results were not significantly different between the derivation and the validation sets.

**Table 9:** Accuracy of MACK-3 for the diagnosis of fibrotic NASH

| | **All** | **Derivation set** | **Validation set** | **p** |
|---|---|---|---|---|
| DA | 93.5 | 93.6 | 93.3 | 0.881 |
| Se | 90.0 | 90.0 | 90.0 | 1.000 |
| Spe | 94.5 | 94.7 | 94.2 | 0.852 |
| NPV | 96.9 | 96.8 | 97.0 | - |
| PPV | 83.4 | 84.2 | 81.8 | - |
| -LR | 0.11 | 0.11 | 0.11 | - |
| +LR | 16.4 | 16.9 | 15.5 | - |
| OR | 155.1 | 160.0 | 146.3 | - |
| LB | 38.4 | 39.6 | 36.0 | 0.356 |
| DA: overall diagnostic accuracy *(i.e.,* rate of well-classified patients, %); Se: sensitivity (%); Spe: specificity (%); NPV: negative predictive value (%); PPV: positive predictive value; -LR: negative likelihood ratio; +LR: positive likelihood ratio; OR: diagnostic odds ratio; LB: liver biopsy requirement (%). | | | | |

Sensitivity analysis

As indicated in **Table 10** below, the accuracy of MACK-3 for the diagnosis of fibrotic NASH was slightly different across the three centers, likely reflecting the different populations of patients they included. To evaluate whether there was a center effect or whether this was due to the different characteristics of the three populations of patients, the influence of center, age, sex, BMI, MetS (metabolic syndrome), oral antidiabetic treatment, insulin treatment, biopsy length $\geq$20mm, NAFLD/bariatric patients and derivation/validation set was tested. By multivariate analysis including all these parameters, only MetS was identified as independently associated with the rate of well-classified patients by MACK-3 **(Table 11)**. Diagnostic accuracy of MACK-3 as a function of MetS presence/absence is presented in **Table 10.**

**Table 10:** Accuracy of MACK-3 for the diagnosis of fibrotic NASH as a function of the center or the presence of metabolic syndrome

| | Center | | | Metabolic syndrome | |
|---|---|---|---|---|---|
| | **Angers** | **Antwerp** | **Nice** | **Absence** | **Presence** |
| AUROC | 0.804±0.025 | 0.815±0.038 | 0.861±0.046 | 0.910±0.042 | 0.795 ± 0.021 |
| DA | 90.8 | 95.4 | 94.7 | 98.0 | 90.9 |
| Se | 93.0 | 78.6 | 95.0 | 91.3 | 89.6 |
| Spe | 89.2 | 98.6 | 94.7 | 98.7 | 91.5 |
| NPV | 94.6 | 96.0 | 99.5 | 99.1 | 95.1 |
| PPV | 86.2 | 91.7 | 61.3 | 87.5 | 82.6 |
| -LR | 0.08 | 0.22 | 0.05 | 0.09 | 0.11 |
| +LR | 8.6 | 56.8 | 18.0 | 70.6 | 10.5 |
| OR | 109.3 | 261.6 | 340.4 | 801.5 | 92.9 |
| LB | 43.8 | 34.7 | 35.6 | 27.1 | 44.9 |

DA: overall diagnostic accuracy (*i.e.,* rate of well-classified patients, %); Se: sensitivity (%); Spe: specificity (%); NPV: negative predictive value (%); PPV: positive predictive value; -LR: negative likelihood ratio; +LR: positive likelihood ratio; OR: diagnostic odds ratio; LB: liver biopsy requirement (%)

**Table 11:** Factors independently associated with correct classification by MACK-3

| Variable | Patients correctly classified by MACK-3 | Patients misdiagnosed by MACK-3 | p | Multivariate analysis | |
|---|---|---|---|---|---|
| | | | | p | OR (95% CI) |
| Age (years) | 47.4 ± 13.6 | 52.5 ± 13.6 | 0.011 | - | - |
| Male sex (%) | 37.9 | 47.2 | 0.191 | - | - |
| Centre (%): | | | 0.057 | - | - |
| *- Angers* | *36.5* | *52.8* | | | |
| *- Antwerp* | 32.6 | 22.6 | | | |
| *- Nice* | *30.9* | 24.5 | | | |
| BMI (kg/m2) | 38.5 ± 7.6 | 36.3 ± 6.9 | 0.029 | - | - |
| MetS (%) | 65.8 | 90.6 | <0.001 | 0.001 | 0.20 (0.08-0.51) |
| Oral antidiabetic treatment (%) | 19.4 | 35.8 | 0.008 | - | - |
| Insulin treatment (%) | 5.7 | 7.5 | 0.540 | - | - |
| Biopsy length ≥ 20mm (%) | 73.2 | 69.2 | 0.521 | - | - |
| Bariatric patients (vs NAFLD, %) | 46.1 | 37.7 | 0.256 | - | - |
| Validation set (%): | 32.9 | 34.0 | 0.881 | - | - |

Practical algorithm for clinical practice

NAFLD represents a very large population in which using MACK-3 to identify the subgroup of patients who need to be treated would require extensive measurement of CK18 and HOMA-IR. To limit unnecessary measurements of these biomarkers, it was evaluated whether simple parameters available in routine practice (age, sex, BMI, MetS, AST, ALT, GGT) can be used in a first step to identify a subgroup with no need for MACK-3 calculation because of a very low prevalence of fibrotic NASH. In the derivation set, multivariate analysis including the seven simple parameters listed above identified AST and MetS (metabolic syndrome) as independent predictors of fibrotic NASH. The best cut-off for AST was calculated at 35 IU/L. 25.1% of the patients had neither MetS nor AST $\geq$ 35 IU/L (also referred to as the "no MetS and AST < 35UI/L" subgroup) and the prevalence of fibrotic NASH in this subgroup was very low (0.7%). This result was confirmed in the validation set where the prevalence of fibrotic NASH was 1.5% in patients having neither MetS nor AST $\geq$ 35UI/L ("no MetS and AST < 35UI/L" subgroup). This very low prevalence was also consistent across the three centers: 0.0% in Angers, 1.3% in Antwerp and 1.0% in Nice (p=0.876).

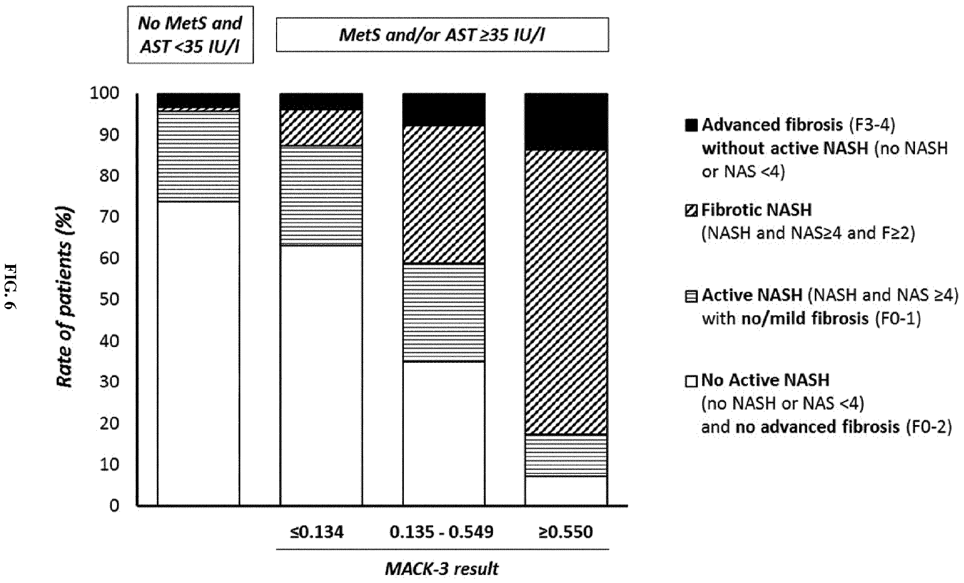

Thus, a new algorithm, referred to as the "MACK-3 algorithm" was derived, according to which MACK-3 is only used for the diagnosis of fibrotic NASH in clinical practice in patients having MetS and/or AST $\geq$ 35 UI/L, as depicted in **Figure 5.** Patients with either MetS or ALT $\geq$ 35 IU/L were stratified according to the three intervals of MACK-3. As shown in **Table 12** below, the accuracy of the MACK-3 algorithm was not significantly different between the derivation and the validation sets. In the whole population, the rates of patients included in the four subgroups of the MACK-3 algorithm were as follow: 25.6% of patients in the "no MetS and AST < 35UI/L" subgroup, 26.1% in the "MetS and/or AST $\geq$ 35 IU/L with MACK-3 $\leq$ 0.134" subgroup, 34.4% of patients in the "MetS and/or AST $\geq$ 35 IU/L with 0.135 $\leq$ MACK-3 $\leq$ 0.549" subgroup, and 13.9% of patients in the "MetS and/or AST $\geq$ 35 IU/L with MACK-3 $\geq$ 0.550" subgroup. The prevalence of fibrotic NASH in the four subgroups of the MACK-3 algorithm was, respectively: 1.0% in the "no MetS and AST < 35UI/L" subgroup, 8.7% in the "MetS and/or AST $\geq$ 35 IU/L with MACK-3 $\leq$ 0.134" subgroup, 33.5% in the "MetS and/or AST $\geq$ 35 IU/l with 0.135 $\leq$ MACK-3 $\leq$ 0.549" subgroup and 69.1% "MetS and/or AST $\geq$ 35 IU/L with MACK-3 $\geq$ 0.550" subgroup. **Figure 6** shows that among the patients included in the last subgroup (MetS and/or AST $\geq$ 35 UI/L with MACK-3 $\geq$ 0.550), 82.7% had fibrotic NASH or advanced F3-4 fibrosis. Only 10.7% of the patients with fibrotic NASH and 15.3% of the patients with advanced F3-4 fibrosis were missed by the MACK-3 algorithm, which respectively represented 2.5% and 2.8% of the whole population. Finally, the MACK-3 algorithm provided an excellent accuracy for the diagnosis of fibrotic NASH with 93.2% well-classified patients, 89.3% sensitivity, 94.4% specificity, 83.1% positive predictive value, and 96.6% negative predictive value as indicated in **Table 12** below.

**Table 12:** Accuracy of the MACK-3 algorithm for the diagnosis of fibrotic NASH

|  | All | Derivation | Validation | p |
|---|---|---|---|---|
| DA | 93.2 | 93.3 | 92.9 | 0.881 |
| Se | 89.3 | 89.1 | 89.8 | 1.000 |
| Spe | 94.4 | 94.6 | 93.9 | 0.710 |
| NPV | 96.6 | 96.5 | 96.8 | - |
| PPV | 83.1 | 83.8 | 81.5 | - |
| -LR | 0.11 | 0.12 | 0.11 | - |
| +LR | 15.8 | 16.5 | 14.7 | - |
| OR | 139.7 | 142.5 | 135.4 | - |
| LB | 34.4 | 35.1 | 32.9 | 0.575 |
| DA: overall diagnostic accuracy (*i.e.,* rate of well-classified patients, %); Se: sensitivity (%); Spe: specificity (%); NPV: negative predictive value (%); PPV: positive predictive value; -LR: negative likelihood ratio; +LR: positive likelihood ratio; OR: diagnostic odds ratio; LB: liver biopsy requirement (%) | | | | |

**Example 2:**

*Patients and methods*

Patients

The study was conducted on the population described in Example 1, obtained by pooling the data from three published studies performed in Angers (Boursier J et al., J Hepatol. 2016 Sep;65(3):570-8), Antwerp (Francque SM et al., Clin Gastroenterol Hepatol 2012;10:1162-8) and Nice (Anty R et al., Aliment Pharmacol Ther 2010;32:1315-22.). Their characteristics are presented hereinabove in Example 1 and detailed in **Table 1.**

The 846 patients were 2:1 randomly divided into a derivation set (564 patients) and a validation set (282 patients), with patient characteristics not being significantly different in the two sets, as shown hereinabove in **Table 6.**

Blood parameters

The data available from the three studies enabled the calculation of the following two non-invasive blood tests: MACK-3 and NASH-score. As described hereinabove, MACK-3 is the combination of the three biomarkers HOMA, AST, and CK-18 in a binary logistic regression. The NASH-score is described in WO2014/049131 and corresponds to the combination of the three biomarkers BMI, CK-18, and hyperglycemia. Both blood tests were performed in the derivation population, in the validation population and in the whole study population.

Statistics

Accuracy of non-invasive tests was evaluated using the Area Under the Receiver Operating Characteristics (AUROC) and compared between tests using the Delong test. Statistical analyses were performed using SPSS version 18.0 software (IBM, Armonk, NY, USA) and SAS 9.1 (SAS Institute Inc., Cary, NC, USA).

*Results*

Comparison of MACK-3 and NASH-score for the diagnosis of NASH

As illustrated below in **Table 13,** the new MACK-3 test, combining HOMA, AST, CK-18 in a binary logistic regression, showed a good accuracy for the non-invasive diagnosis of NASH with AUROC at $0.785 \pm 0.028$ in the validation set. By contrast, the NASH-score combining BMI, CK-18, and hyperglycemia showed a poor accuracy with AUROC at $0.497 \pm 0.035$ in the validation set. Similar results were obtained in the derivation set and in the whole study population.

Thus, MACK-3 showed a significantly greater accuracy than NASH-score for the diagnosis of NASH.

**Table 13:** AUROC of MACK-3 and NASH-score for the diagnosis of NASH

|  | Population | | |
|---|---|---|---|
|  | All (n=846) | Derivation (n=564) | Validation (n=282) |
| MACK-3 | $0.723 \pm 0.018$ | $0.699 \pm 0.022$ | $0.785 \pm 0.028$ |
| NASH-score | $0.498 \pm 0.020$ | $0.498 \pm 0.025$ | $0.497 \pm 0.035$ |
| p | $< 0.001$ | $< 0.001$ | $< 0.001$ |

Comparison of MACK-3 and NASH-score for the diagnosis of fibrotic NASH

As observed in Example 1 and in **Table 14** below, MACK-3 showed a very good accuracy for the non-invasive diagnosis of fibrotic NASH with AUROC at $0.847 \pm 0.030$ in the validation set. By contrast, NASH-score showed a moderate accuracy with AUROC at $0.636 \pm 0.042$ in the validation set. Similar results were obtained in the derivation set and in the whole study population.

Thus, MACK-3 showed a significantly greater accuracy than NASH-score for the diagnosis of fibrotic NASH.

**Table 14:** AUROC of MACK-3 and NASH-score for the diagnosis of fibrotic NASH

|  | Population | | |
|---|---|---|---|
|  | All (n=846) | Derivation (n=564) | Validation (n=282) |
| MACK-3 | 0.846 ± 0.016 | 0.845 ± 0.019 | 0.847 ± 0.030 |
| NASH-score | 0.647 ± 0.023 | 0.652 ± 0.028 | 0.636 ± 0.042 |
| p | < 0.001 | < 0.001 | < 0.001 |

**Example 3:**

***Patients and methods***

Patients

The study was conducted on the population described in Example 1, obtained by pooling the data from three published studies performed in Angers (Boursier J et al., J Hepatol. 2016 Sep;65(3):570-8), Antwerp (Francque SM et al., Clin Gastroenterol Hepatol 2012;10:1162-8) and Nice (Anty R et al., Aliment Pharmacol Ther 2010;32:1315-22). Their characteristics are presented hereinabove in Example 1 and detailed in **Table 1.**

The 846 patients were 2:1 randomly divided into a derivation set (564 patients) and a validation set (282 patients), with patient characteristics not being significantly different between the two sets, as shown hereinabove in **Table 6.**

Blood parameters

The data available from the three studies enabled the calculation of the following non-invasive blood tests: MACK-3 and combinations A, B, and C. As described hereinabove, MACK-3 is the combination of the three biomarkers HOMA, AST, and CK-18 in a binary logistic regression. The combination A corresponds to the combination of CK-18, BMI, and HOMA. The combinations B and C are described in WO2014/049131 and corresponds to the combinations of:

- combination B: BMI, CK-18 and glycemia; and
- combination C: CK-18, weight and insulin.

All blood tests were performed in the derivation population, in the validation population and in the whole study population.

Statistics

Accuracy of non-invasive tests was evaluated using the Area Under the Receiver Operating Characteristics (AUROC) and compared between tests using the Delong test. Statistical analyses were performed using SPSS version 18.0 software (IBM, Armonk, NY, USA) and SAS 9.1 (SAS Institute Inc., Cary, NC, USA).

***Results***

Comparison of MACK-3 and combinations A, B and C for the diagnosis of fibrotic NASH

As observed in Example 1 and in **Table 15** below, MACK-3 showed a very good accuracy for the non-invasive diagnosis of fibrotic NASH with AUROC at 0.847 ± 0.030 in the validation set. Furthermore, MACK-3 showed a significantly higher accuracy than the combinations A, B and C previously described in WO2014/049131, both in the derivation and in the validation sets.

**Table 15:** AUROC of MACK-3 and combinations A, B and C for the diagnosis of fibrotic NASH

|  | Population | | |
|---|---|---|---|
|  | All (n=846) | Derivation (n=564) | Validation (n=282) |
| MACK-3 | 0.846 ± 0.016 | 0.845 ± 0.019 | 0.847 ± 0.030 |

(continued)

| | Population | | |
|---|---|---|---|
| | All (n=846) | Derivation (n=564) | Validation (n=282) |
| Combination A | 0.757 ± 0.021 | 0.766 ± 0.025 | 0.740 ± 0.037 |
| Combination B | 0.784 ± 0.019 | 0.798 ± 0.022 | 0.757 ± 0.038 |
| Combination C | 0.736 ± 0.022 | 0.747 ± 0.025 | 0.717 ± 0.040 |
| MACK-3 vs (p): | | | |
| Combination A | < 0.001 | < 0.001 | < 0.001 |
| Combination B | < 0.001 | < 0.040 | 0.001 |
| Combination C | < 0.001 | < 0.001 | < 0.001 |

In conclusion, the results of Tables 14 and 15 demonstrate than the MACK-3 test, combining HOMA, AST, and CK-18 in a binary logistic regression, is characterized by a significantly greater diagnostic performance for the diagnosis of fibrotic NASH than several of the combinations of markers described in WO2014/049131.

**Example 4:**

***Patients and methods***

Patients

The study was conducted on the population described in Example 1, obtained by pooling the data from three published studies performed in Angers (Boursier J et al., J Hepatol. 2016 Sep;65(3):570-8), Antwerp (Francque SM et al., Clin Gastroenterol Hepatol 2012;10:1162-8) and Nice (Anty R et al., Aliment Pharmacol Ther 2010;32:1315-22). Their characteristics are presented hereinabove in Example 1 and detailed in **Table 1.**
The 846 patients were 2:1 randomly divided into a derivation set (564 patients) and a validation set (282 patients), with patient characteristics not being significantly different between the two sets, as shown hereinabove in **Table 6.**

Blood parameters

The data available from the three studies enabled the calculation of the following non-invasive blood tests: MACK-3 and the CHA index. As described hereinabove, MACK-3 is the combination of the three biomarkers HOMA, AST, and CK-18 in a binary logistic regression. The CHA index is determined and used as described in Polyzos SA et al. Ann Hepatol. 2013 Sep-Oct;12(5):749-57, and corresponds to the following formula: CK-18 x HOMA x AST)/1000.
All blood tests were performed in the derivation population, in the validation population and in the whole study population.

Statistics

Accuracy of non-invasive tests was evaluated using the Area Under the Receiver Operating Characteristics (AUROC) and compared between tests using the Delong test. Statistical analyses were performed using SPSS version 18.0 software (IBM, Armonk, NY, USA) and SAS 9.1 (SAS Institute Inc., Cary, NC, USA).

***Results***

Comparison of MACK-3 and the CHA index for the diagnosis of fibrotic NASH

**Table 16** below compares the performance of MACK-3 and the CHA index for the diagnosis of fibrotic NASH in both the derivation and the validation sets. As shown below, the overall diagnostic accuracy of MACK-3 is significantly higher than that of the CHA index both in the derivation set (93.6% of correctly classified patients vs 76.6% of correctly classified patients respectively, p < 0.001) and in the validation set (93.3% of correctly classified patients vs 75.3% of correctly classified patients, respectively, p < 0.001). In particular, when compared to the CHA index, MACK-3 is characterized by a significantly higher sensitivity (78.3% vs 90%, respectively, in the validation set, p = 0.016) and a significantly higher specificity (74.4% vs 94.2%, respectively, in the validation set, p < 0.001). These results demonstrate that with MACK-3, the proportion of patients correctly diagnosed either as having fibrotic NASH or as not having fibrotic NASH is significantly higher than with the CHA index.

**Table 16:** Comparison of the diagnostic performances of MACK-3 and the CHA index for the diagnosis of fibrotic NASH

|  | Derivation set | | | Validation set | | |
|---|---|---|---|---|---|---|
|  | MACK-3 | CHAI [a] | p | MACK-3 | CHAI [a] | p |
| DA | 93.6 | 76.6 | <0.001 | 93.3 | 75.3 | <0.001 |
| Se | 90.0 | 78.5 | <0.001 | 90.0 | 78.3 | 0.016 |
| Spe | 94.7 | 76.0 | <0.001 | 94.2 | 74.4 | <0.001 |
| NPV | 96.8 | 91.8 | - | 97.0 | 92.2 | - |
| PPV | 84.2 | 50.7 | - | 81.8 | 47.0 | - |
| -LR | 0.11 | 0.28 | - | 0.11 | 0.29 | - |
| +LR | 16.9 | 3.3 | - | 15.5 | 3.1 | - |
| OR | 160.0 | 11.6 | - | 146.3 | 10.5 | - |

[a] determined according to the methods described in Polyzos SA et al. Ann Hepatol. 2013 Sep-Oct;12(5):749-57. DA: overall diagnostic accuracy (*i.e.,* rate of well-classified patients, %); Se: sensitivity (%); Spe: specificity (%); NPV: negative predictive value (%); PPV: positive predictive value; -LR: negative likelihood ratio; +LR: positive likelihood ratio; OR: diagnostic odds ratio

In conclusion, the results of Table 16 demonstrate that the MACK-3 test, combining HOMA, AST, and CK-18 in a binary logistic regression, is characterized by a significantly greater diagnostic performance for the diagnosis of fibrotic NASH than the CHA index as described in Polyzos SA et al. Ann Hepatol. 2013 Sep-Oct;12(5):749-57.

**Claims**

1. A method for assessing the presence and/or the severity of fibrotic non-alcoholic steatohepatitis (fibrotic NASH) in a subject, wherein said method comprises:

    - measuring cytokeratin 18 (CK-18), aspartate aminotransferase (AST) and Homeostasis model assessment of insulin resistance (HOMA) in a sample of said subject;
    - combining the three obtained measures in a binary logistic regression to obtain a score value ranging from 0 to 1; and
    - comparing the score value to at least one predetermined reference value, wherein said predetermined reference value is derived from the determination of the score in one or more subjects who are identified as suffering from non-alcoholic fatty liver disease (NAFLD), NASH, or fibrotic NASH,

    thereby assessing the presence and/or the severity of fibrotic NASH in the subject.

2. The method according to claim **1,** wherein said method is computerized.

3. The method according to claim **1** or **2,** wherein the score value is compared to two predetermined reference values.

4. The method according to claim **3,** wherein the first predetermined reference value is an exclusion cut-off, preferably ranging from about 0.1 to about 0.2.

5. The method according to claim **3** or **4,** wherein the second predetermined reference value is an affirmation cut-off, preferably ranging from about 0.5 to about 0.6.

6. The method according to any one of claims **1** to **5,** wherein the sample is a serum sample, a plasma sample or a blood sample.

7. The method according to any one of claims **1** to **6,** wherein the subject is an animal, preferably a human.

8. The method according to any one of claims **1** to **7,** wherein the subject has a preexisting disease or condition, preferably metabolic syndrome and/or non-alcoholic fatty liver disease (NAFLD).

9. The method according to any one of claims **1** to **8,** wherein the subject has been previously diagnosed with non-alcoholic fatty liver disease (NAFLD), and wherein said method comprises the additional step of first assessing the presence of metabolic syndrome in the subject suffering from NAFLD and measuring the level of aspartate aminotransferase (AST) in a sample of said subject suffering from NAFLD before performing the method according to any one of claims **1** to **8.**

10. A method for monitoring the progression of fibrotic non-alcoholic steatohepatitis (fibrotic NASH) in a subject, wherein said method comprises:

> a) determining a score value, said score value ranging from 0 to 1, by measuring in a sample of said subject cytokeratin 18 (CK-18), aspartate aminotransferase (AST) and Homeostasis model assessment of insulin resistance (HOMA), and combining the three obtained measures in a binary logistic regression; and
> b) comparing the score value to a personalized reference value.

11. The method according to claim **10,** wherein the personalized reference value for a subject is a score value previously determined for said subject.

12. The method according to claim **10** or claim **11,** wherein a score value is determined at least twice over a period of time.

13. A method for monitoring treatment response in a subject treated for fibrotic non-alcoholic steatohepatitis (fibrotic NASH), wherein said method comprises:

> - determining a score value, said score value ranging from 0 to 1, before the beginning of the treatment by measuring in a sample of said subject cytokeratin 18 (CK-18), aspartate aminotransferase (AST) and Homeostasis model assessment of insulin resistance (HOMA), and combining the three obtained measures in a binary logistic regression;
> - repeating the determination of the score value after the beginning of the treatment; and
> - comparing the score values obtained before and after the beginning of the treatment,

> thereby determining whether said treatment is effective.

14. The method according to claim **13,** wherein the determination of the score value after the beginning of the treatment is repeated at least twice, and preferably at regular intervals.

**Patentansprüche**

1. Verfahren zum Bewerten des Vorhandenseins und/oder der Schwere von fibrotischer nicht-alkoholischer Steatohepatitis (fibrotischer NASH) bei einem Patienten, wobei das Verfahren umfasst:

> - Messen von Cytokeratin 18 (CK-18), Aspartat-Aminotransferase (AST) und HOMA (Homöstase-Modell-Bewertung der Insulinresistenz) in einer Probe des Patienten;
> - Kombinieren der drei erhaltenen Messwerte in einer binären logistischen Regression, um einen Score-Wert im Bereich von 0 bis 1 zu erhalten; und
> - Vergleichen des Score-Wertes mit mindestens einem zuvor bestimmten Referenzwert, wobei der zuvor bestimmte Referenzwert von der Bestimmung des Scores bei einem oder mehreren Patienten abgeleitet wird, die als solche identifiziert werden, die an einer nicht-alkoholischen Fettlebererkrankung (NAFLD), NASH oder fibrotischen NASH leiden,

> dadurch das Vorhandensein und/oder die Schwere der fibrotischen NASH bei einem Patienten bewertet wird.

2. Verfahren nach Anspruch **1**, wobei das Verfahren computergestützt ist.

3. Verfahren nach Anspruch **1** oder **2**, wobei der Score-Wert mit zwei zuvor bestimmten Referenzwerden verglichen wird.

4. Verfahren nach Anspruch **3**, wobei der erste zuvor bestimmte Referenzwert ein Ausgrenzungsausschnitt ist, der vorzugsweise im Bereich von etwa 0,1 bis etwa 0,2 liegt.

5. Verfahren nach Anspruch **3** oder **4**, wobei der zweite zuvor bestimmte Referenzwert ein Bestätigungsausschnitt ist, der vorzugsweise im Bereich von etwa 0,5 bis etwa 0,6 liegt.

6. Verfahren nach einem der Ansprüche **1** bis **5**, wobei die Probe eine Serumprobe, eine Plasmaprobe oder eine Blutprobe ist.

7. Verfahren nach einem der Ansprüche **1** bis **6**, wobei der Patient ein Tier, vorzugsweise ein Mensch ist.

8. Verfahren nach einem der Ansprüche **1** bis **7**, wobei der Patient eine bereits vorliegende Erkrankung oder Bedingung, vorzugsweise ein metabolisches Syndrom und/oder eine nicht-alkoholische Fettlebererkrankung (NAFLD) aufweist.

9. Verfahren nach einem der Ansprüche **1** bis **8**, wobei dem Patienten zuvor eine nicht-alkoholische Fettlebererkrankung (NAFLD) diagnostiziert wurde, und wobei das Verfahren den zusätzlichen Schritt einer ersten Bewertung des Vorhandenseins eines metabolischen Syndroms bei dem Patienten, der an NAFLD leidet, und das Messen des Niveaus an Aspartat-Aminotransferase (AST) in einer Probe des Patienten, der an NAFLD leidet, vor dem Ausführen des Verfahrens nach einem der Ansprüche **1** bis **8** umfasst.

10. Verfahren zum Überwachen des Fortschritts einer fibrotischen nicht-alkoholischen Steatohepatitis (fibrotischen NASH) bei einem Patienten, wobei das Verfahren umfasst:

    a) Bestimmen eines Score-Wertes, wobei der Score-Wert in einem Bereich von 0 bis 1 liegt, durch Messen in einer Probe des Patienten von Cytokeratin 18 (CK-18), Aspartat-Aminotransferase (AST) und HOMA (Homöstase-Modell-Bewertung der Insulinresistenz), und Kombinieren der drei erhaltenen Messwerte in einer binären logistischen Regression; und
    b) Vergleichen des Score-Wertes mit einem personenspezifischen Referenzwert.

11. Verfahren nach Anspruch **10**, wobei der personenspezifische Referenzwert für einen Patienten ein zuvor für den Patienten bestimmter Score-Wert ist.

12. Verfahren nach Anspruch **10** oder Anspruch **11**, wobei ein Score-Wert mindestens zwei Mal über einen Zeitraum hinweg bestimmt wird.

13. Verfahren zum Überwachen einer Behandlungsreaktion bei einem Patienten, der wegen einer fibrotischen nicht-alkoholischen Steatohepatitis (fibrotischen NASH) behandelt wird, wobei das Verfahren umfasst:

    - Bestimmen eines Score-Werts, wobei der Score-Wert in einem Bereich von 0 bis 1 liegt, vor dem Beginnen der Behandlung durch Messen in einer Probe des Patienten von Cytokeratin 18 (CK-18), Aspartat-Aminotransferase (AST) und HOMA (Homöstase-Modell-Bewertung der Insulinresistenz), und Kombinieren der drei erhaltenen Messwerte in einer binären logistischen Regression;
    - Wiederholen der Bestimmung des Score-Wertes nach dem Beginnen der Behandlung; und
    - Vergleichen der Score-Werte, die vor und nach dem Beginnen der Behandlung erhalten werden,

    dadurch Bestimmen, ob die Behandlung wirksam ist.

14. Verfahren nach Anspruch **13**, wobei das Bestimmen des Score-Wertes nach dem Beginnen der Behandlung mindestens zwei Mal, und vorzugsweise in regelmäßigen Intervallen wiederholt wird.

**Revendications**

1. Une méthode pour évaluer la présence et/ou la sévérité d'une stéatohépatite non alcoolique fibrosante (NASH fibrosante) chez un sujet, dans laquelle ladite méthode comprend :

   - mesurer la cytokératine 18 (CK-18), l'aspartate aminotransférase (AST) et HOMA (modèle d'évaluation de l'homéostasie de la résistance à l'insuline) dans un échantillon dudit sujet ;
   - combiner les trois mesures obtenues dans une régression logistique binaire pour obtenir une valeur de score allant de 0 à 1 ; et
   - comparer la valeur de score à au moins une valeur de référence prédéterminée, dans laquelle ladite valeur de référence prédéterminée est dérivée de la détermination du score chez un ou plusieurs sujets qui sont identifiés comme souffrant d'une stéatose hépatique non alcoolique (NAFLD), d'une NASH ou d'une NASH fibrosante,

   évaluant ainsi la présence et/ou la sévérité d'une NASH fibrosante chez le sujet.

2. La méthode selon la revendication **1,** dans laquelle ladite méthode est informatisée.

3. La méthode selon la revendication **1** ou **2,** dans laquelle la valeur de score est comparée à deux valeurs de référence prédéterminées.

4. La méthode selon la revendication **3,** dans laquelle la première valeur de référence prédéterminée est un seuil d'exclusion, de préférence allant d'environ 0,1 à environ 0,2.

5. La méthode selon la revendication **3** ou **4,** dans laquelle la seconde valeur de référence prédéterminée est un seuil d'affirmation, de préférence allant d'environ 0,5 à environ 0,6.

6. La méthode selon l'une quelconque des revendications **1** à **5,** dans laquelle l'échantillon est un échantillon de sérum, un échantillon de plasma ou un échantillon de sang.

7. La méthode selon l'une quelconque des revendications **1** à **6,** dans laquelle le sujet est un animal, de préférence un humain.

8. La méthode selon l'une quelconque des revendications **1** à **7,** dans laquelle le sujet a une maladie ou condition préexistante, de préférence un syndrome métabolique et/ou une stéatose hépatique non alcoolique (NAFLD).

9. La méthode selon l'une quelconque des revendications **1** à **8,** dans laquelle le sujet a été précédemment diagnostiqué avec une stéatose hépatique non alcoolique (NAFLD), et dans laquelle ladite méthode comprend l'étape additionnelle de premièrement évaluer la présence d'un syndrome métabolique chez le sujet souffrant de NAFLD et mesurer le niveau d'aspartate aminotransférase (AST) dans un échantillon dudit sujet souffrant de NAFLD avant d'effectuer la méthode selon l'une quelconque des revendications **1** à **8.**

10. Une méthode pour surveiller la progression d'une stéatohépatite non alcoolique fibrosante (NASH fibrosante) chez un sujet, dans laquelle ladite méthode comprend :

    a) déterminer une valeur de score, ladite valeur de score allant de 0 à 1, en mesurant dans un échantillon dudit sujet la cytokératine 18 (CK-18), l'aspartate aminotransférase (AST) et HOMA (modèle d'évaluation de l'homéostasie de la résistance à l'insuline), et en combinant les trois mesures obtenues dans une régression logistique binaire ; et
    b) comparer la valeur de score à une valeur de référence personnalisée.

11. La méthode selon la revendication **10,** dans laquelle la valeur de référence personnalisée pour un sujet est une valeur de score précédemment déterminée pour ledit sujet.

12. La méthode selon la revendication **10** ou la revendication **11,** dans laquelle une valeur de score est déterminée au moins deux fois sur une période de temps.

13. Une méthode pour surveiller la réponse au traitement chez un sujet traité pour une stéatohépatite non alcoolique fibrosante (NASH fibrosante), dans laquelle ladite méthode comprend :

- déterminer une valeur de score, ladite valeur de score allant de 0 à 1, avant le début du traitement en mesurant dans un échantillon dudit sujet la cytokératine 18 (CK-18), l'aspartate aminotransférase (AST) et HOMA (modèle d'évaluation de l'homéostasie de la résistance à l'insuline), et en combinant les trois mesures obtenues dans une régression logistique binaire ;
- répéter la détermination de la valeur de score après le début du traitement ; et
- comparer les valeurs de score obtenues avant et après le début du traitement, déterminant ainsi si ledit traitement est efficace.

14. La méthode selon la revendication 13, dans laquelle la détermination de la valeur de score après le début du traitement est répétée au moins deux fois, et de préférence à intervalles réguliers.

Fibrosis stage (n)

| | | F0 | F1 | F2 | F3 | F4 |
|---|---|---|---|---|---|---|
| No NASH (n=389) | NAS <4 (n=368) | 75 | 108 | 158 | 21 | 6 |
| | NAS ≥4 (n=21) | 2 | 7 | 8 | 3 | 1 |
| NASH (n=457) | NAS <4 (n=81) | 17 | 23 | 18 | 19 | 4 |
| | NAS ≥4 (n=376) | 99 | 80 | **103** | **84** | **10** |

All (n=846)

Fibrotic NASH

**FIG. 1**

A

Proportion of all fibrotic NASH patients (%)

FIB4
<1.30: lower interval
1.30 – 2.67: grey zone
>2.67: higher interval

NAFLD fibrosis score
< -1.455: lower interval
-1.455 – 0.676: grey zone
>0.676: higher interval

Lower interval: 49,0 / 30,4
Grey zone: 40,2 / 51,5
Higher interval: 10,8 / 18,0

B

Prevalence of fibrotic NASH (%)

FIB4
<1.30: lower interval
1.30 – 2.67: grey zone
>2.67: higher interval

NAFLD fibrosis score
< -1.455: lower interval
-1.455 – 0.676: grey zone
>0.676: higher interval

Lower interval: 15,5 / 14,4
Grey zone: 42,2 / 30,6
Higher Interval: 55,3 / 38,0

**FIG. 2**

FIG. 3A

FIG. 3B

FIG. 4

**FIG. 5**

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2490026 A
- WO 2013049509 A
- US 2013183687 A
- WO 2014049131 A
- WO 2015192854 A

### Non-patent literature cited in the description

- **CHALASANI N et al.** *Gastroenterology,* 2012, vol. 142, 1592-609
- **KLEINER DE et al.** *Hepatology,* 2005, vol. 41, 1313-21
- **BRUNT EM et al.** *Hepatology,* 2011, vol. 53, 810-20
- **ANGULO P et al.** *Hepatology,* 2007, vol. 45, 846-54
- **RUDWILL et al.** *Liver Int.,* June 2015, vol. 35 (6), 1700-6
- **POLYZOS SA et al.** *Ann Hepatol.,* September 2013, vol. 12 (5), 749-57
- **HAMZA et al.** *Horm Res Paediatr.,* 2016, vol. 86 (1), 11-20
- **CHALASANI et al.** *Hepatology,* 2012, vol. 55 (6), 2005-2023
- **MATTHEWS et al.** *Diabetologia,* 1985, vol. 28, 412-419
- **KATZ A et al.** *J Clin Endocrinol Metab,* 2000, vol. 85, 2402-2410
- **FORTHOFER RN ; LEE ES ; HERNANDEZ M.** Biostatistics: A guide to Design, Analysis, and Discovery. Elsevier, 2006
- EASL-EASD-EASO Clinical Practice Guidelines for the management of non-alcoholic fatty liver disease. *J Hepatol,* 2016, vol. 64, 1388-1402
- **YOUNOSSI ZM et al.** *Hepatology,* 2016, vol. 64, 73-84
- **SHAH AG et al.** *Clin Gastroenterol Hepatol,* 2009, vol. 7, 1104-12
- **BOURSIER J et al.** *J Hepatol.,* September 2016, vol. 65 (3), 570-8
- **FRANCQUE SM et al.** *Clin Gastroenterol Hepatol,* 2012, vol. 10, 1162-8
- **ANTY R et al.** *Aliment Pharmacol Ther,* 2010, vol. 32, 1315-22
- **ALBERTI KG et al.** *Circulation,* 2009, vol. 120, 1640-1645
- **SANYAL AJ et al.** *Hepatology,* 2011, vol. 54, 344-53
- **SANYAL AJ et al.** *Hepatology,* 2015, vol. 61, 1392-405
- **HARRISON SA et al.** *Gut,* 2008, vol. 57, 1441-7
- **STERLING RK et al.** *Hepatology,* 2006, vol. 43, 1317-25
- **CALES P et al.** *Clin Biochem,* 2008, vol. 41, 10-18
- **SHROUT PE ; FLEISS JL.** *Psychol Bull,* 1979, vol. 86, 420-428
- **KLEINER DE et al.** *Hepatology,* 2005, vol. 41, 1313-1321